# EUROPEAN PATENT APPLICATION

(11) **EP 4 344 739 A2**
(43) Date of publication of application: **03.04.2024**
(21) Application number: 23205669.7
(22) Date of filing: 28.06.2019
(51) Int. Cl.: A61P 11/00

(54) **THERAPEUTIC LUNG REPAIR BY INHALATION OF LUNG SPHEROID CELL-SECRETED FACTORS**

(30) Priority: 29.06.2018 US 201862691811 P
(62) Divisional of application: 19826230.5
(71) Applicant: North Carolina State University, Raleigh, NC 27606 (US)
(72) Inventor: CHENG, Ke, Raleigh, 27606 (US); DINH, Phuong-Uyen, Raleigh, 27606 (US)
(74) Representative: Berggren Oy

(57) **Abstract**

Idiopathic pulmonary fibrosis (IPF) is currently the most fatal form of idiopathic interstitial lung disease in which persistent lung injuries result in scar tissue formation. Provided are methods and compositions for the treatment of pulmonary conditions such as fibrosis. Lung spheroid cell-derived conditioned media (LSC-CM) and exosomes (LSC-EXO) are used to treat different models of lung injury. Inhalation treatment with LSC-CM and LSC-EXO-derived compositions can attenuate and/or reverse bleomycin- and silica-induced fibrosis, reestablish normal alveolar structure and decrease extracellular matrix accumulation.

## Description

### CROSS-REFERENCE TO RELATED APPLICATIONS

This application claims priority to and the benefit of U.S. Provisional Application 62/691,811 titled "THERAPEUTIC LUNG REPAIR BY INHALATION OF LUNG SPHEROID CELL-SECRETED FACTORS" filed June 29, 2018, the entire disclosure of which is incorporated herein by reference in its entirety.

### TECHNICAL FIELD

The present disclosure is generally related to methods of treatment of a lung pathological condition responsive to a stem cell-derived secretome or fraction thereof.

### BACKGROUND

The entire human body and all its organs are covered with a protective layer of epithelial cells. The epithelial cells in the lungs not only facilitate oxygen exchange but also defends against continuous exposure to inhaled irritants and toxins every day. Fortunately, lungs have the ability to undergo facultative regeneration because of the resident stem and progenitor cell populations that are essential for maintaining the tissue homeostasis and repair in response to acute and/or chronic lung injuries.

However, respiratory-related morbidity and mortality are on the rise. Some chronic lung injuries have been linked to environmental factors or lifestyle choices such as smoking. Idiopathic Pulmonary Fibrosis (IPF) is a progressive and fatal form of interstitial lung disease, characterized by fibroblastic foci, alveolar honeycombing and persistent and unremitting fibrosis that eventually leads to the destruction of lung architecture and ultimately complete respiratory failure. With the exact pathological mechanism(s) and cause of IPF unknown, the disease has poor prognosis. There remain few treatment options for IPF. To date, only two Food and Drug Administration (FDA) approved therapies exist, pirfenidone and nintedanib, yet these treatments are only palliative and merely delay disease progression. They do not halt or reverse the fibrosis that is already established.

As new treatment strategies continue to evolve, cell-based therapies have emerged as a promising option with multiple clinical trials utilizing stem cells. Although stem cells have beneficial effects, their clinical applications face many challenges including extensive labor, high cost, and safety concerns. Cell-based therapy carries a certain risk of tumorigenicity and immunogenicity, along with cell stability concerns, as stem and progenitor cells all have an inherent risk of transformation during prolonged in-vitro cell cultures. Also, cell therapy products need to be carefully preserved and processed before clinical applications. Cumulating evidence indicates that the regenerative ability of adult stem cells is primarily due to their paracrine activity. Therefore, utilization of the stem cell secretome or stem cell conditioned media (CM) in place of the actual cells could mitigate not only these limitations but retain the regenerative benefits of cell-based therapies.

### SUMMARY

One aspect of the disclosure encompasses embodiments of a pharmaceutical composition comprising a lung spheroid cell-conditioned medium or a lung spheroid cell-secreted therapeutic agent, or a population of lung spheroid cell-derived exosomes in an amount effective in modulating a pulmonary pathological condition when delivered to an animal or human subject in need thereof.

In some embodiments of this aspect of the disclosure, the pulmonary pathological condition is pulmonary lung fibrosis.

In some embodiments of this aspect of the disclosure, the pharmaceutical composition can comprise at least one of an isolated polypeptide, an isolated peptide, or an isolated nucleic acid, and wherein the isolated polypeptide, isolated peptide, or isolated nucleic acid is secreted by a cultured lung spheroid cell.

In some embodiments of this aspect of the disclosure, the pharmaceutical composition can comprise a population of lung spheroid cell-derived exosomes isolated from a lung spheroid cell-conditioned medium.

In some embodiments of this aspect of the disclosure, the nucleic acid can be an miRNA.

In some embodiments of this aspect of the disclosure, the pharmaceutical composition administered to the respiratory tract of the animal or human subject can further comprise a pharmaceutically acceptable carrier.

In some embodiments of this aspect of the disclosure, the pharmaceutical composition can be a lyophilized conditioned cell culture medium generated by culturing a population of lung spheroid cells therein.

In some embodiments of this aspect of the disclosure, the pharmaceutical composition administered to the respiratory tract of an animal or human subject by the nebulizer can be a lyophilized powder.

Another aspect of the disclosure encompasses embodiments of a method of producing a pharmaceutical composition comprising a lung spheroid cell-conditioned medium or a lung spheroid cell-secreted therapeutic agent, or a population of lung spheroid cell-derived exosomes, the method comprising the steps of: (a) obtaining a population of isolated pulmonary stem cells from a pulmonary tissue of an animal or human subject; (b) generating a conditioned cell culture medium by culturing the isolated population of stem cells in a cell culture medium; and (c) removing the cultured cells from the conditioned culture medium.

In some embodiments of this aspect of the disclosure, the method can further comprise the step of isolating the conditioned cell culture medium at least one of (a) at least one of an isolated polypeptide, an isolated peptide, or an isolated nucleic acid secreted by a cultured lung spheroid cell into the conditioned cell culture, or (b) a population of lung spheroid cell-derived exosomes generated by a population cultured lung spheroid cell into the conditioned cell culture medium.

In some embodiments of this aspect of the disclosure, the method can further comprise the step of lyophilizing the conditioned culture medium.

In some embodiments of this aspect of the disclosure, the method can further comprise the step of lyophilizing the at least one of (a) at least one of an isolated polypeptide, an isolated peptide, or an isolated nucleic acid secreted by a cultured lung spheroid cell into the conditioned cell culture, or (b) a population of lung spheroid cell-derived exosomes generated by a population cultured lung spheroid cell into the conditioned cell culture medium.

In some embodiments of this aspect of the disclosure, the method the lyophilized product of the method can be combined with a pharmaceutically acceptable medium.

Yet another aspect of the disclosure encompasses embodiments of a method of treating a pathological condition of an animal or human pulmonary system, wherein the method comprises administering to a region of a the respiratory tract of an animal or human subject a pharmaceutical composition comprising a lung spheroid cell-conditioned medium or a lung spheroid cell-secreted therapeutic agent, or a population of lung spheroid cell-derived exosomes in an amount effective in modulating a pulmonary pathological condition when delivered to the animal or human subject in need thereof.

In some embodiments of this aspect of the disclosure, the pulmonary pathological condition is pulmonary fibrosis.

In some embodiments of this aspect of the disclosure, the pharmaceutical composition can comprise at least one of an isolated polypeptide, an isolated peptide, or an isolated nucleic acid, and wherein the isolated polypeptide, isolated peptide, or isolated nucleic acid is secreted by a cultured lung spheroid cell.

In some embodiments of this aspect of the disclosure, the pharmaceutical composition can comprise a population of lung spheroid cell-derived exosomes isolated from a lung spheroid cell-conditioned medium.

In some embodiments of this aspect of the disclosure, the nucleic acid can be an miRNA.

In some embodiments of this aspect of the disclosure, the pharmaceutical composition administered to the respiratory tract of the animal or human subject can further comprise a pharmaceutically acceptable carrier.

In some embodiments of this aspect of the disclosure, the pharmaceutical composition can be a lyophilized conditioned cell culture medium generated by culturing a population of lung spheroid cells therein.

In some embodiments of this aspect of the disclosure, the pharmaceutical composition administered to the respiratory tract of an animal or human subject by the nebulizer can be a lyophilized powder.

### BRIEF DESCRIPTION OF THE DRAWINGS

Further aspects of the present disclosure will be more readily appreciated upon review of the detailed description of its various embodiments, described below, when taken in conjunction with the accompanying drawings.
Figs. 1A-1I illustrate that stem cell conditioned media reverses alveolar epithelial cell damage caused by chronic Bleomycin injury.
Fig. 1A illustrates a schematic of the conditioned media procedure and the experimental study schematic; n = 6-7 per group.
Fig. 1B illustrates a macroscopic view of the explanted lungs at study endpoint.
Fig. 1C and 1D illustrate representative Tunel staining of apoptotic cells for each treatment group (Fig. 1C) and quantification of percent of Tunel positive cells (Fig. 1D); Scale bar = 100µm *P ≤ 0.05; each dot represents data from one animal; n = 6.
Fig. 1E illustrates a representative H&E staining. Top: Scale bar = 500µm Bottom: Scale bar = 200µm.
Fig. 1F illustrates a quantification of fibrosis by Ashcroft score; *P ≤ 0.05; ***P ≤ 0.001; each dot represents data from one animal; n = 7.
Fig. 1G illustrates representative Gomori's trichrome staining muscle fibers, collagen, nuclei, and erythrocytes. Scale bar = 1000µm.
Fig. 1H illustrates a representative picrosirius staining of collagen types I and III. Scale bar = 50µm.
Fig. 1I illustrates quantification of pulmonary hydroxyproline levels; *P ≤ 0.05; ***P ≤ 0.001; each dot represents data from one animal; n = 6.
Figs 2A-2G illustrate that LSC-CM inhalation treatment promotes alveolar repair.

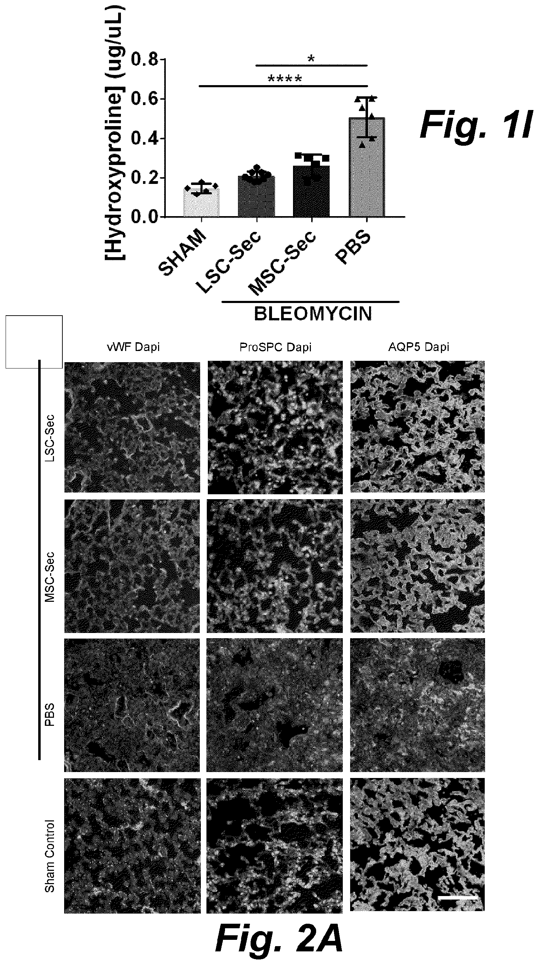

Fig. 2A illustrates representative immunostaining of lung sections for von Willebrand Factor (vWF), pro-surfactant protein C (Pro-SPC) and aquaporin 5 (AQP5). Scale bar = 100µm.

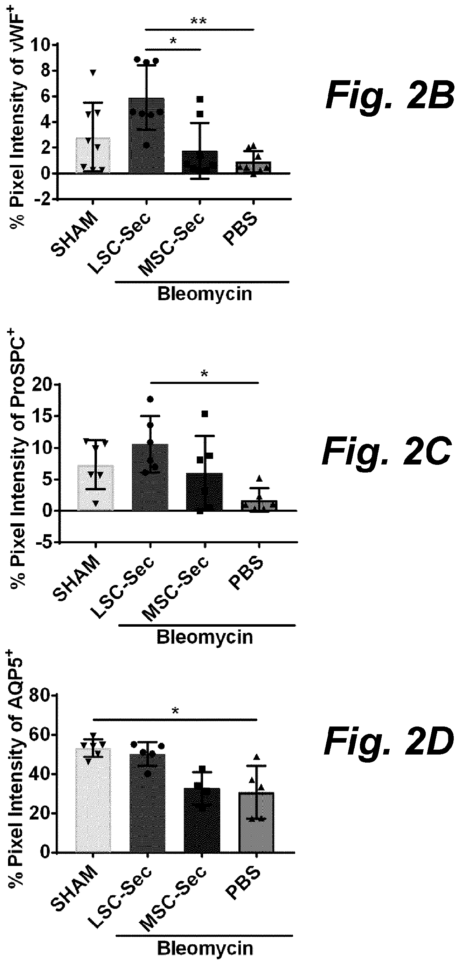

Figs. 2B-2D illustrate quantification of percent pixel intensity of vWF+ (Fig. 2B), ProSPC+ (Fig. 2C), and AQP5+ (Fig. 2D). *P ≤ 0.05; **P ≤ 0.01; each dot represents data from one animal; n = 4.

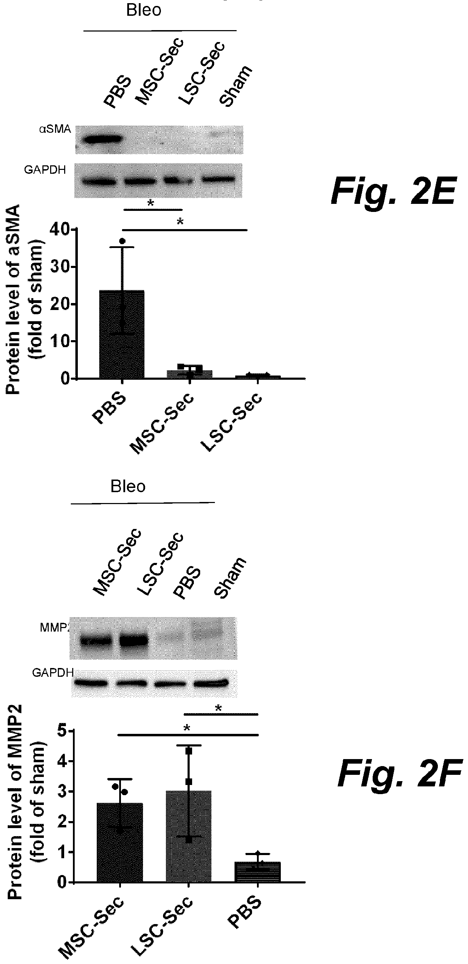

Figs. 2E-2F illustrate Western blot analysis of alpha smooth muscle actin (αSMA) (Fig. 2E), matrix metalloproteinase 2 (MMP2) (Fig. 2F), and GAPDH loading control with corresponding quantification of protein levels as fold of sham control; *P ≤ 0.05; each dot represents data from one animal; n = 3.

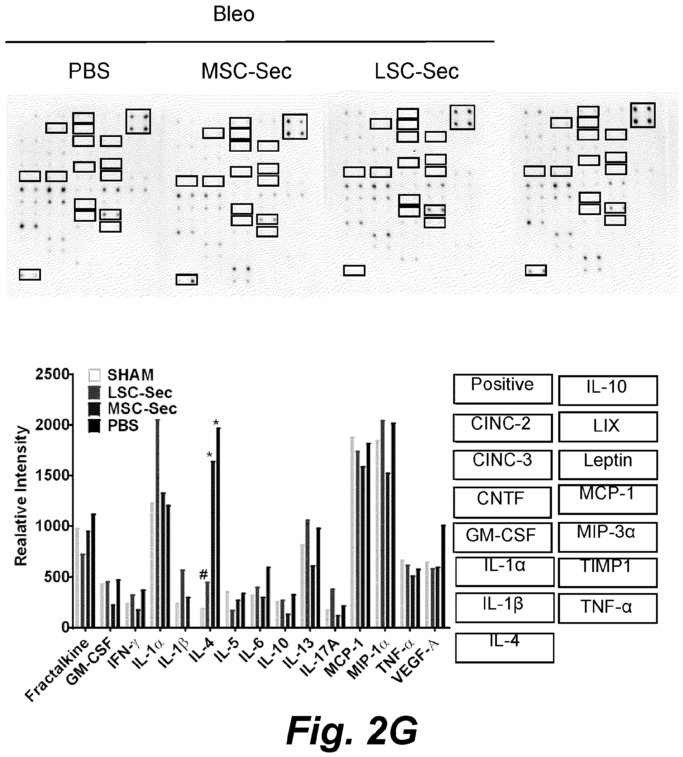

Fig. 2G illustrates a representative cytokine array with quantification of relative intensity; * P ≤ 0.05 compared to sham; # P ≤ 0.05 compared to PBS.

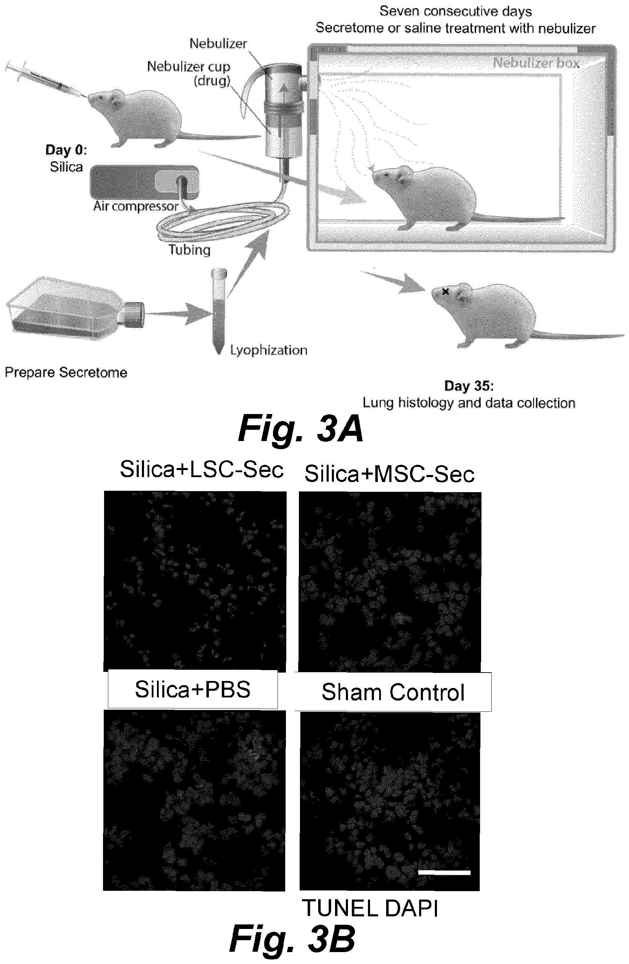

Figs. 3A-3K illustrate lung repair and fibrosis in mice after silica-injury.
Fig. 3A illustrates an experimental study schematic of the silica induced fibrosis study in A/J mice; n = 10 per group.
Fig. 3B illustrates representative Tunel staining of apoptotic cells for each treatment group and quantification of percent of Tunel positive cells; Scale bar = 50µm; each dot represents data from one animal; n = 5.

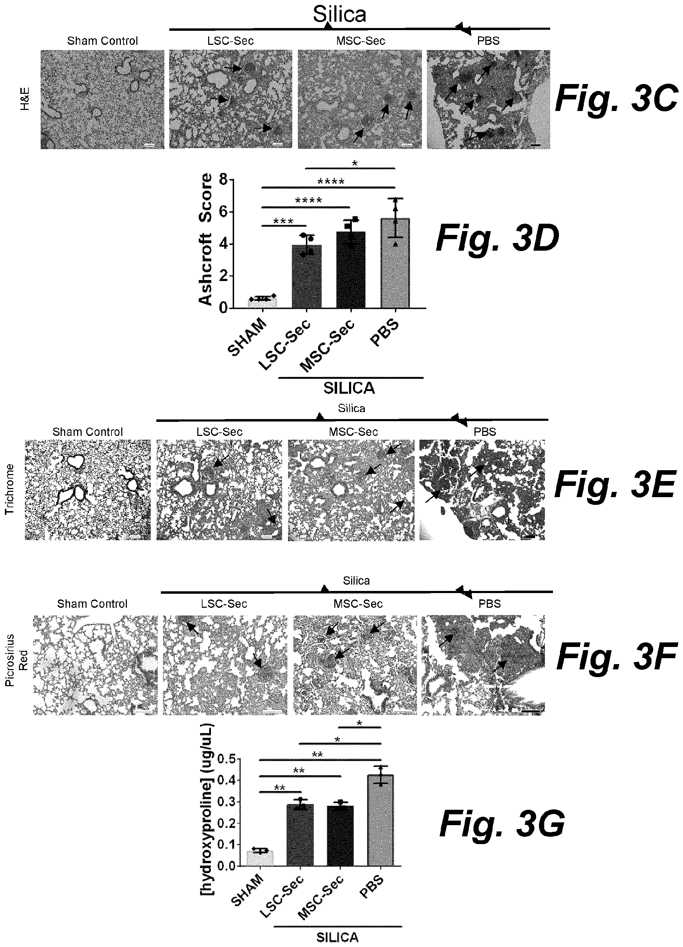

Fig. 3C illustrates a representative H&E staining. Scale bar = 100µm
Fig. 3D illustrates quantification of fibrosis by Ashcroft score; *P ≤ 0.05; ***P ≤ 0.001; ****P ≤ 0.0001; each dot represents data from one animal; n = 4.
Fig. 3E illustrates Gomori's trichrome staining of muscle fibers, collagen, nuclei, and erythrocytes. Scale bar = 100µm.
Fig. 3F illustrates picrosirius staining of collagen types I and III. Scale bar = 100µm.
Fig. 3G illustrates quantification of pulmonary hydroxyproline levels; *P ≤ 0.05; each dot represents data from one animal; n = 4.

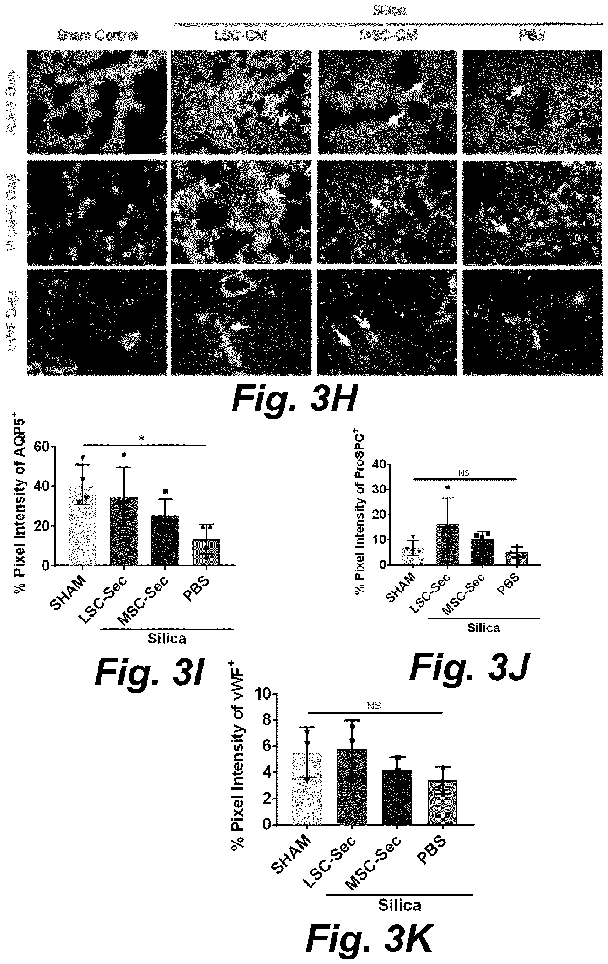

Fig. 3H illustrates a representative immunostaining of lung sections for aquaporin 5 (AQP5), pro-surfactant protein C (Pro-SPC) and von Willebrand Factor (vWF). Scale bar = 50µm.
Figs. 3I-3K illustrate the quantification of percent pixel intensity of AQP5+ (Fig. 3I), ProSPC+ (Fig. 3J), and vWF+ (Fig. 3K). *P ≤ 0.05; each dot represents data from one animal; n = 4.

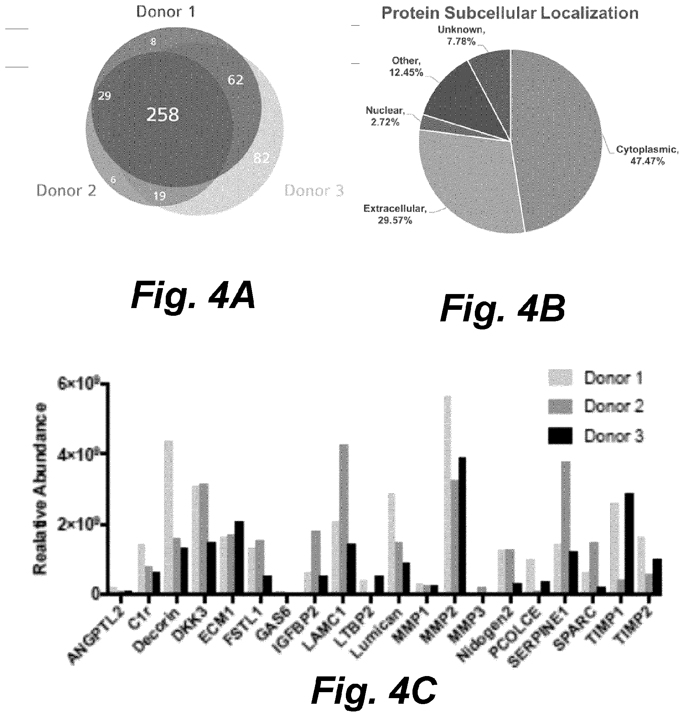

Figs. 4A-4D illustrate a proteomic analysis of LSC-CM.
Fig. 4A illustrates a Venn diagram of proteins identified in three LSC-CM.
Fig. 4B illustrates a pie chart of protein subcellular location of all common proteins in all three LSC-CM.
Fig. 4C illustrates a relative abundance of 20 of the 103 extracellular proteins identified in all three LSC-CM.

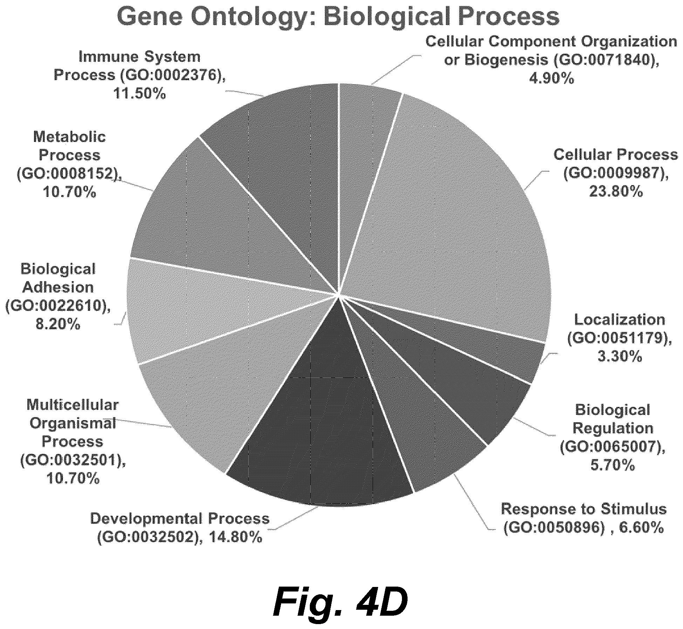

Fig. 4D illustrates a gene ontology pie chart of biological process associated with the shared extracellular proteins.

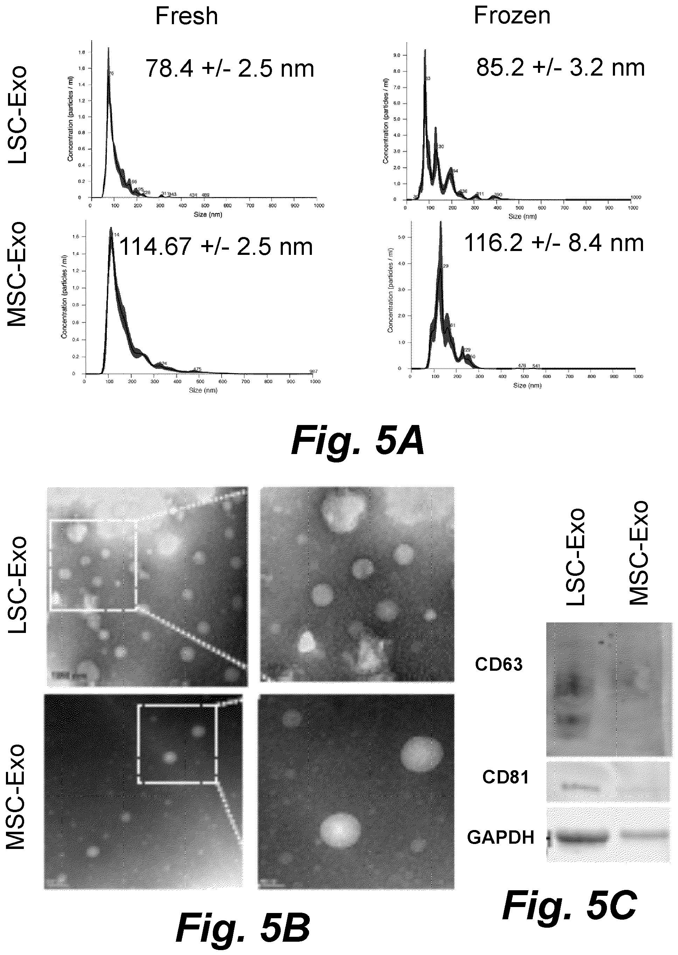

Figs. 5A-5L illustrate the therapeutic potential of exosome inhalation treatment in rats post-Bleo injury.
Fig. 5A illustrates a size analysis of fresh and frozen exosome particles by NTA NanoSight.
Fig. 5B illustrates a representative transmission electron micrograph (TEM) of LSC-EXO. Left scale bar = 0.2µm. Right scale bar = 50nm.
Fig. 5C illustrates a Western blot analysis of CD63, CD81 and GAPDH protein in LSC- and MSC-EXO.

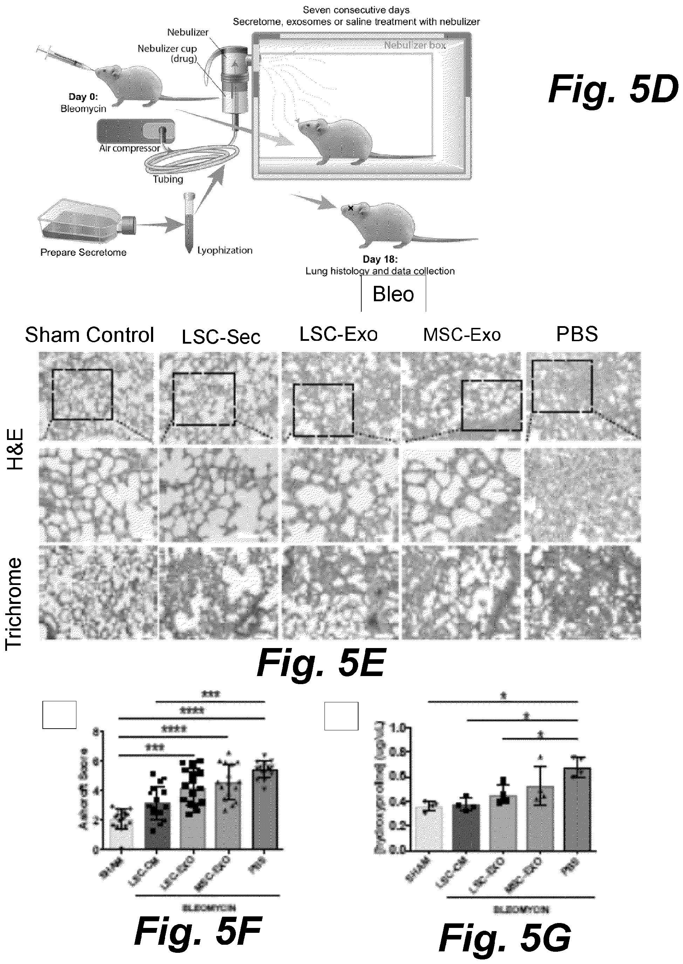

Fig. 5D illustrates an experimental study schematic of the exosome study in SD rats; n = 12 per group.
Fig. 5E illustrates a representative H&E staining Top: Scale bar = 100µm Bottom: Scale bar = 50µm and Gomori's trichrome staining. Scale bar = 100µm (bottom); muscle fibers, collagen, nuclei, and erythrocytes.
Fig. 5F illustrates a quantification of fibrosis by Ashcroft score; Scale bar = µm ***P ≤ 0.001; ****P ≤ 0.0001; each dot represents data from one animal; n = 12.
Fig. 5G illustrates a quantification of pulmonary hydroxyproline levels; *P ≤ 0.05; each dot represents data from one animal; n = 4.

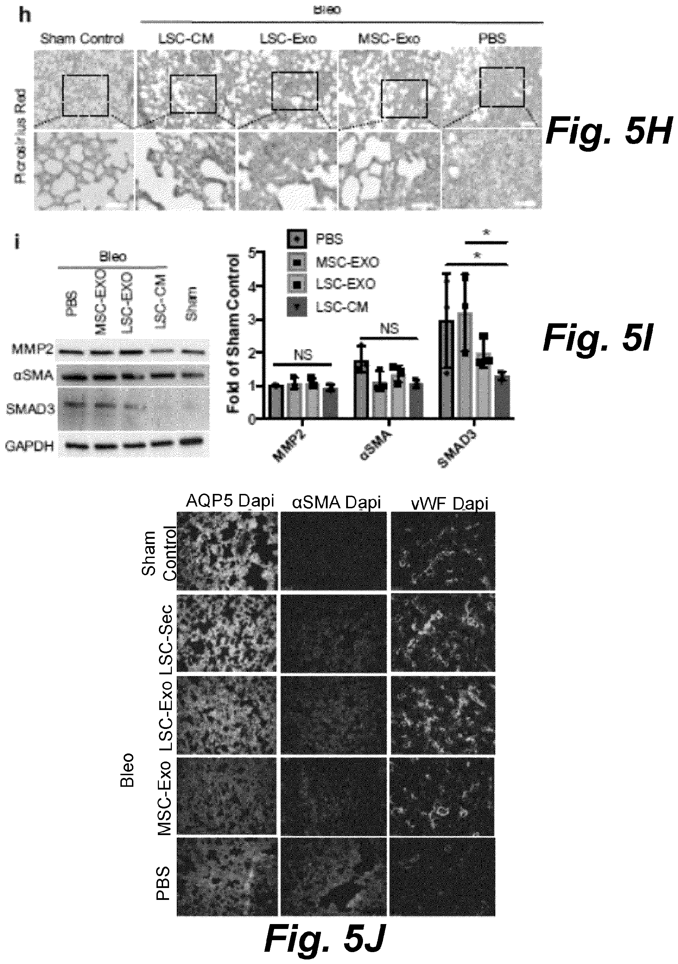

Fig. 5H illustrates a representative picrosirius staining of Collagen types I and III; Scale bar = 50µm.
Fig. 5I illustrates a Western blot analysis of matrix metalloproteinase 2 (MMP2), alpha smooth muscle actin (αSMA), SMAD3 and GAPDH loading control.
Fig. 5J illustrates a representative immunostaining of lung sections for aquaporin 5 (AQP5), alpha- smooth muscle actin (αSMA), and von Willebrand Factor (vWF). Scale bar = 75µm.

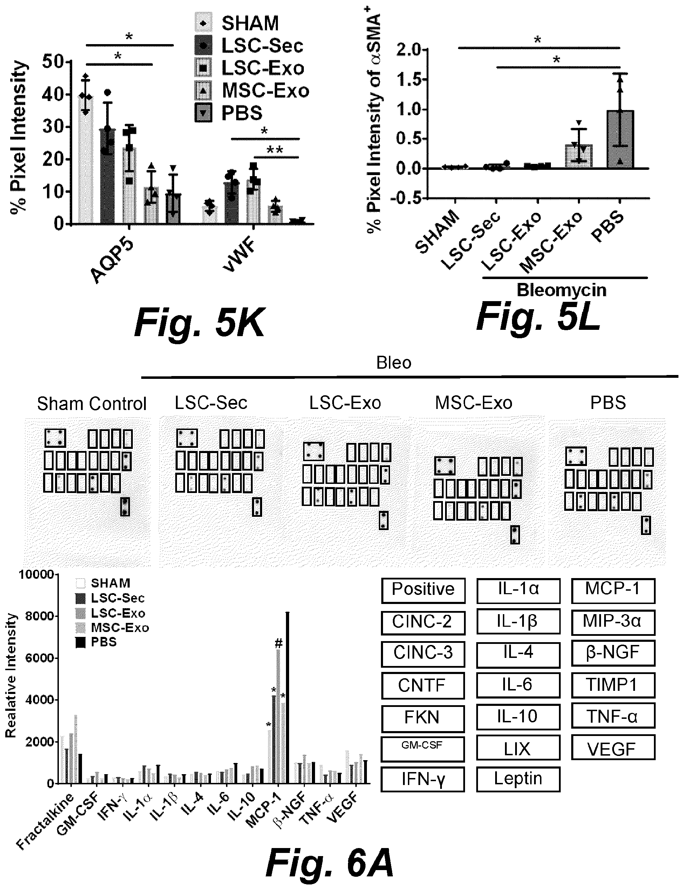

Fig. 5K illustrates a quantification of pixel intensity of AQP5 and vWF. *P ≤ 0.05; **P ≤ 0.01; each dot represents data from one animal; n = 4.
Fig. 5L illustrates a quantification of pixel intensity of αSMA. *P ≤ 0.05; each dot represents data from one animal; n = 4.
Figs. 6A-6K illustrate exosome treatment improves pulmonary function post-Bleo and exosome miRNA profiling.

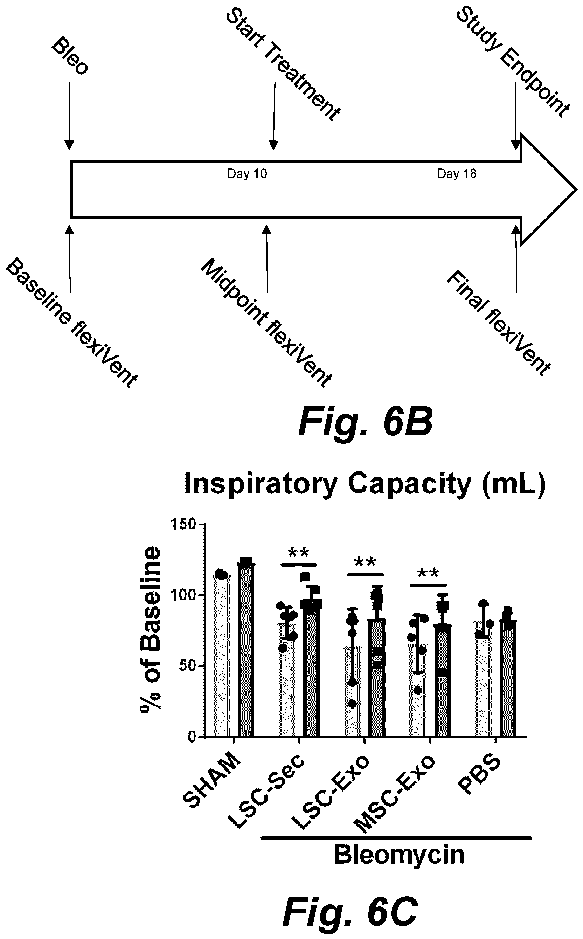

Fig. 6A illustrates a representative rat cytokine array detecting 19 rat proteins from blood serum. *P ≤ 0.05 from PBS group; # P ≤ 0.05 from Sham group Fig. 6B illustrates a schematic of pulmonary function measurements.

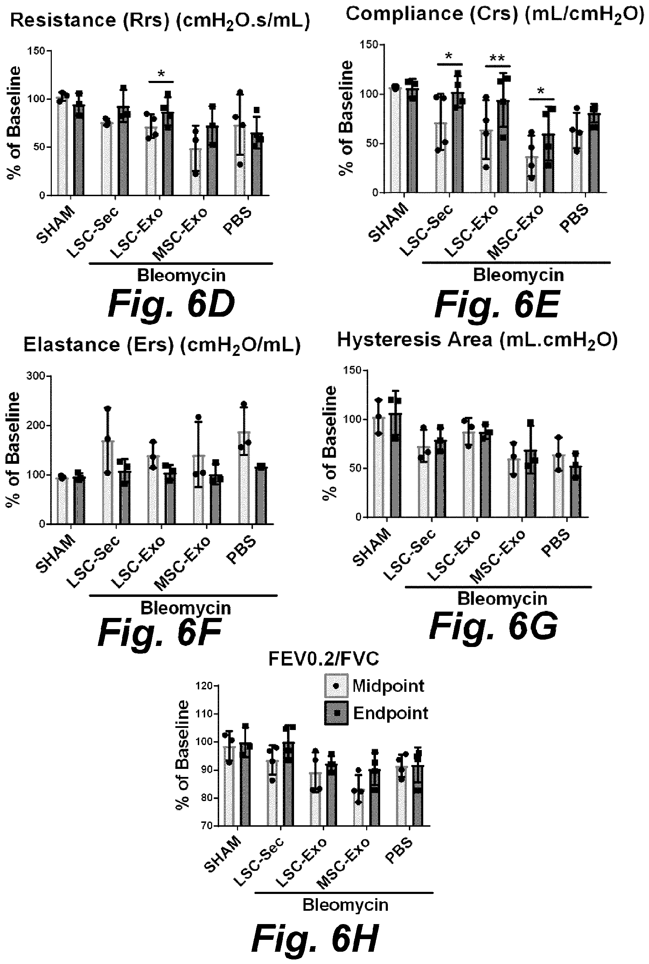

Figs. 6C-6G illustrate quantification of lung function measurements; *P ≤ 0.05; **P ≤ 0.01. (Fig. 6C) pulmonary inspiratory capacity; (Fig. 6D); pulmonary resistance (Fig. 6E) pulmonary compliance; (Fig. 6F) pulmonary elastance; (Fig. 6G) hysteresis area.
Fig. 6H illustrates a forced expiratory volume (FEV) to forced vital capacity (FVC) ratio.

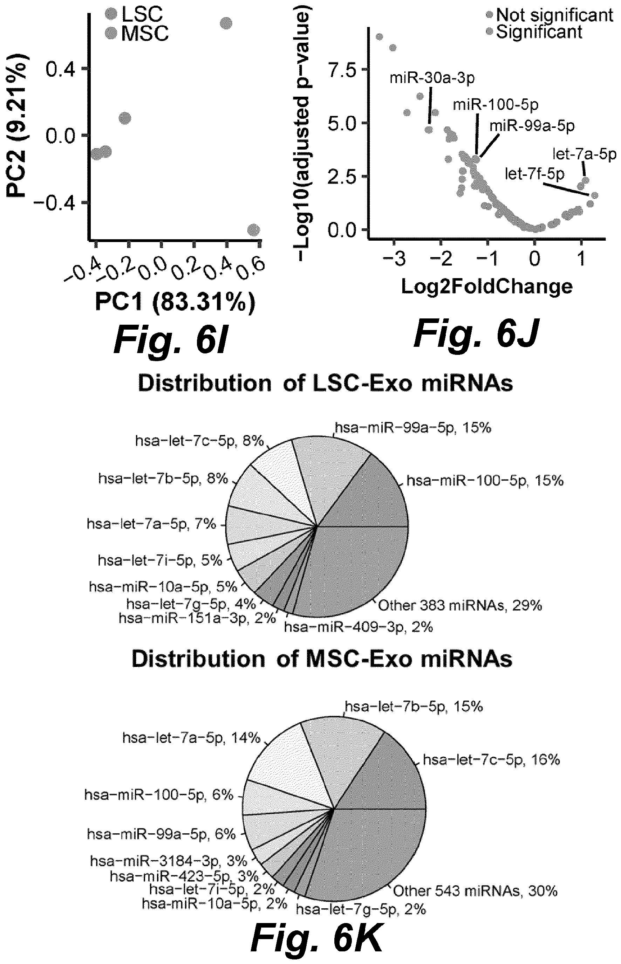

Fig. 6I illustrates a principal component analysis chart of LSC- and MSC-exosome microRNA content.
Fig. 6J illustrates a Volcano plot of differentially expressed miRNA of LSC- and MSC-exosomes miRNA content.
Fig. 6K illustrates a distribution of the top 10 miRNAs in LSC- exosomes (top) and MSC-exosomes (bottom).

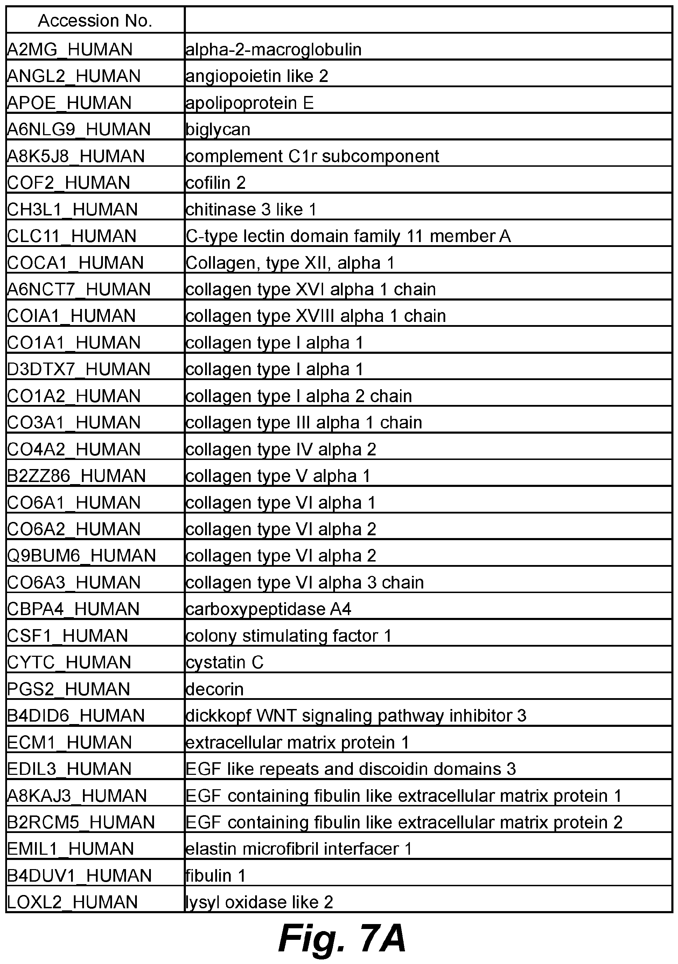

Figs. 7A-7C illustrate the proteins found in three different cultured lung spheroid stem cell secretomes of conditioned culture medium.

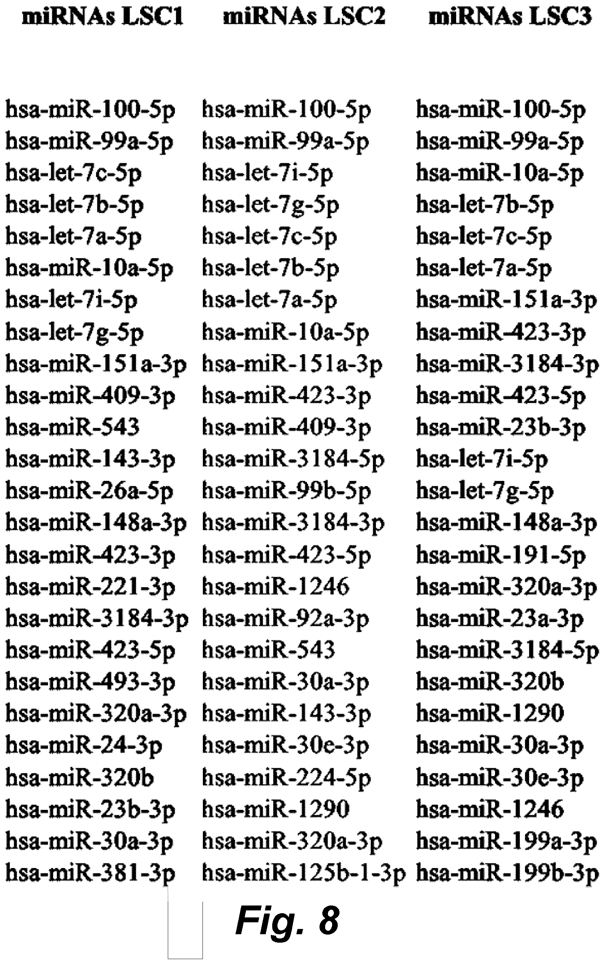

Fig. 8 illustrates the identities of miRNAs found in the conditioned medium of three populations of cultured lung spheroid cell lines.

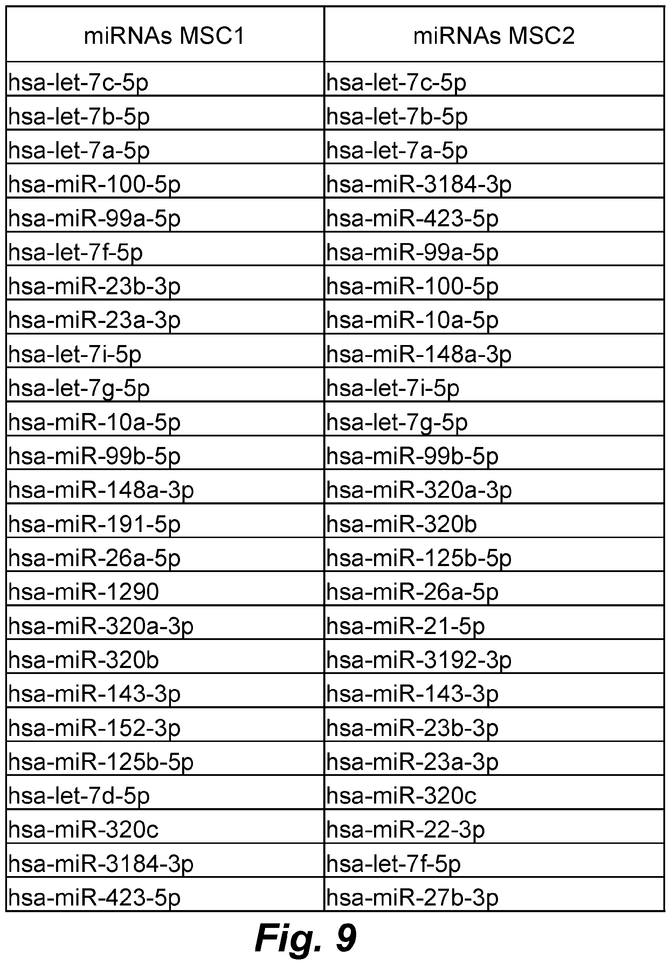

Fig. 9 illustrates the identities of miRNAs found in the conditioned medium from two populations of cultured mesenchymal stem cell lines.

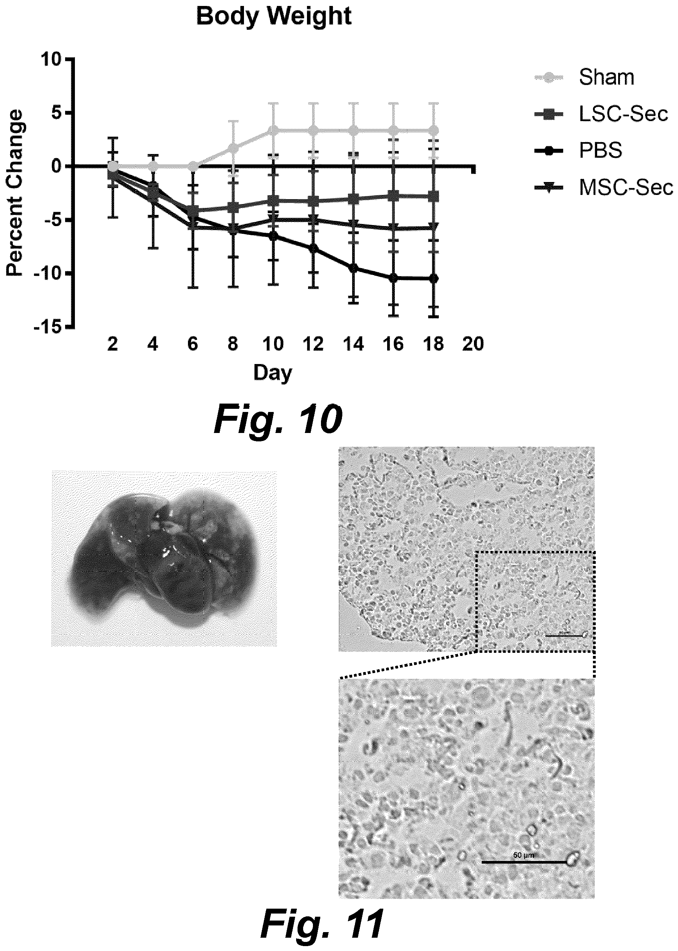

Fig. 10 illustrates changes in body weight after delivery of lung spheroid cell conditioned medium to bleomycin-treated mice.
Fig. 11 illustrates confirmation of the efficacy of nebulization to deliver an aerosolized liquid labeled with methylene blue dye to mice lungs.

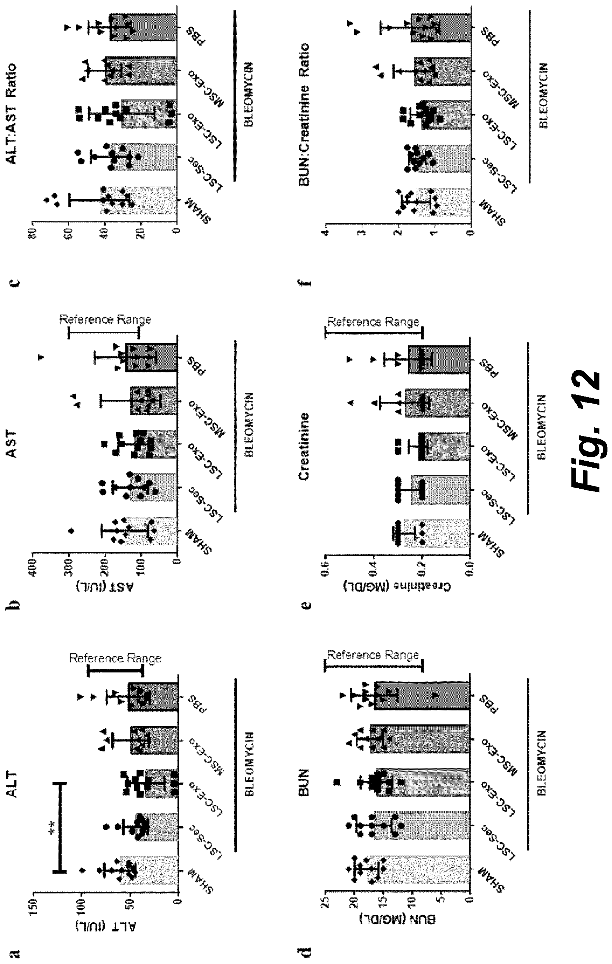

Fig. 12 illustrates a series of graphs showing that conditioned medium or exosome treatment did not alter kidney and liver function.

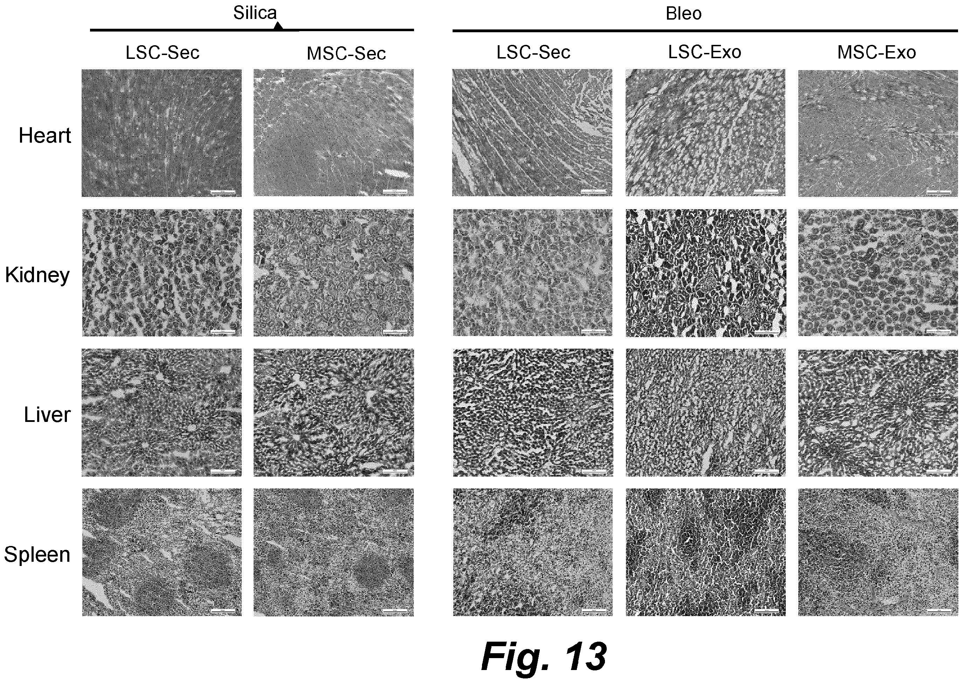

Fig. 13 illustrates a histological analysis of organs of treated animals in both Bleo and silica studies not showing damage or toxicity due to exposure to conditioned culture medium or exosomes derived from lung spheroid cells.

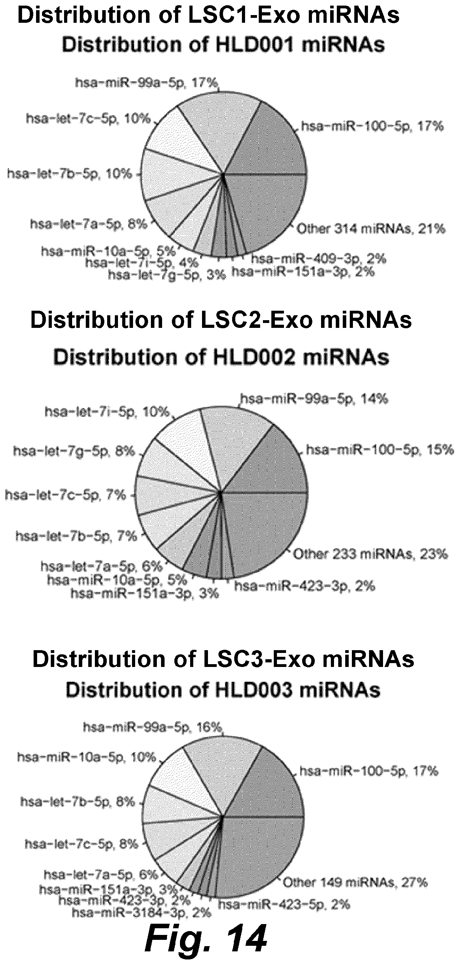

Fig. 14 illustrates a series of pie charts showing the distribution of miRNA species in the exosomes derived from three populations of lung spheroid cells.

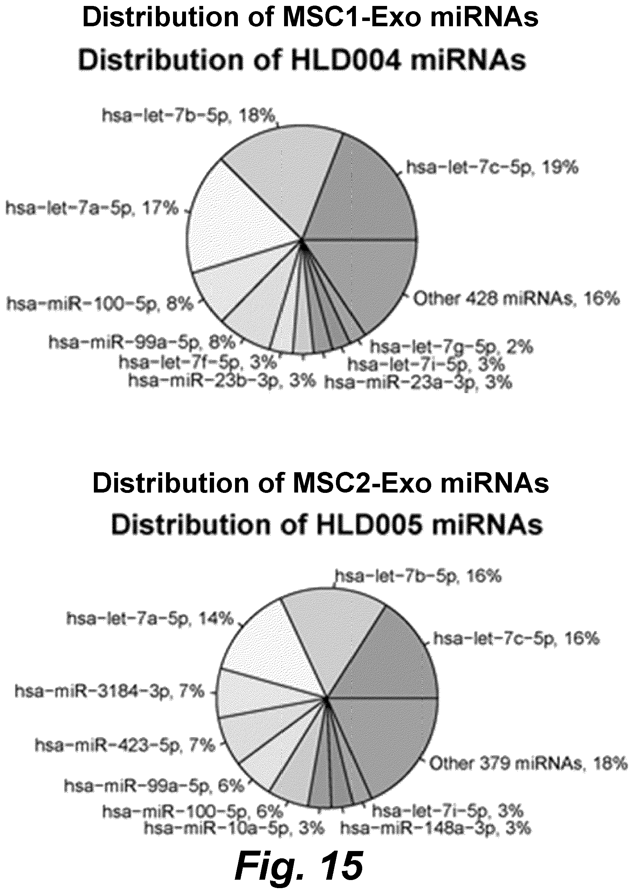

Fig. 15 illustrates a series of pie charts showing the distribution of miRNA species in the exosomes derived from two populations of mesenchymal stem cells.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of medicine, organic chemistry, biochemistry, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

As used herein, the following terms have the meanings ascribed to them unless specified otherwise. In this disclosure, "comprises," "comprising," "containing" and "having" and the like can have the meaning ascribed to them in U.S. patent law and can mean " includes," "including," and the like; "consisting essentially of" or "consists essentially" or the like, when applied to methods and compositions encompassed by the present disclosure refers to compositions like those disclosed herein, but which may contain additional structural groups, composition components or method steps (or analogs or derivatives thereof as discussed above). Such additional structural groups, composition components or method steps, etc., however, do not materially affect the basic and novel characteristic(s) of the compositions or methods, compared to those of the corresponding compositions or methods disclosed herein.

Prior to describing the various embodiments, the following definitions are provided and should be used unless otherwise indicated.

Numerical ranges recited herein by endpoints include all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about." "About" as used herein indicates that a number, amount, time, etc., is not exact or certain but reasonably close to or almost the same as the stated value. Therefore, the term "about" means plus or minus 0.1 to 50%, 5-50%, or 10-40%, preferably 10-20%, more preferably 10% or 15%, of the number to which reference is being made. Further, it is to be understood that "a", "an," and "the" include plural referents unless the content clearly dictates otherwise. Thus, for example, reference to a composition comprising "a compound" includes a mixture of two or more compounds.

### Abbreviations

αSMA, alpha smooth muscle actin; ANGPTL2, angiopoietin-like protein 2; AT1, alveolar type 1 epithelial cells; AT2, alveolar type 2 epithelial cells; AQP5, aquaporin 5; Bleo, bleomycin, ; C1r, complement component 1r; CM, conditioned media; Crs, compliance; DDK3, dickkopf-related protein 3; DNA, deoxyribonucleic acid; ECM, extracellular matrix; Ers, elastance; EV, extracellular vesicles; EXO, exosomes, ; FDA, food and drug administrations; FEV, forced expiratory volume ; FSTL1, follistatin like 1; FVC, forced vital capacity; GAS6, growth arrest-specific 6; H&E, hematoxylin and eosin; IC, inspiratory capacity; IGFBP2, insulin like growth factor binding protein 2; IL, interleukin; IPF, idiopathic pulmonary fibrosis; i.t., intratracheal; LSC, lung spheroid cells; MCP-1, monocyte chemoattractant protein 1; miR, micro RNA; MMP, matrix metalloproteinase; MSC, mesenchymal stem cells; PCOLCE, procollagen C-endopeptidase enhancer 1; ProSPC, prosurfactant protein C; RNA, ribonucleic acid; Rrs, resistance; SERPINE1, serpine family E member 1; SMAD3, mothers against decapentaplegic homolog 3; SPARC, secreted proteins acidic and rich in cysteine; TIMP, tissue inhibitor of metalloproteinases; vWF, von Willebrand factor;

### Definitions

The terms "administration of" and "administering" a compound or composition as used herein refers to providing a composition of the disclosure to the individual in need of treatment. The composition of the present disclosure may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration.

The term "allogeneic" refers to deriving from or originating in another subject or patient. An "allogeneic transplant" refers to collection (e.g., isolation) and transplantation of the cells or organs from one subject into the body of another. In some instances, an "allogeneic transplant" includes cells grown or cultured from another subject's cells.

The term "autologous" refers to deriving from or originating in the same subject or patient. An "autologous transplant" refers to collection (e.g., isolation) and re-transplantation of a subject's own cells or organs. In some instances, an "autologous transplant" includes cells grown or cultured from a subject's own cells. For example, in the methods of the present disclosure, the cardiac stem cells may be derived from a cardiac tissue sample excised from the heart of the patient to be treated, cultured, engineered to be fused with platelet membrane vesicles according to the methods of the disclosure and then administered to the same patient for the treatment of a cardiovascular injury therein.

The term "biocompatible" as used herein refers to a material that does not elicit any undesirable local or systemic effects *in vivo.*

The terms "biological material" and "biological tissue" as used herein refer to cells or tissue *in vivo* (e.g. cells or tissue of a subject) and *in vitro* (e.g. cultured cells).

The term "biomolecule species" as used herein refers to any molecule that may be of biological origin and/or interact with a cell in contact therewith. A biomolecule species of use in the microparticles of the disclosure may be, but are not to be limited to, a protein, a polypeptide, a peptide, a nucleic acid molecule, a saccharide, a polysaccharide, a cytokine and the like that may be, but is not limited to, increasing or decreasing the proliferation of the cell or cell line, may sustain viability and/or proliferation of the cell or cell line, or may initiate a change in the cell type from a stem cell type, a precursor cell type or a progenitor cell type.

The term "cell" as used herein refers to an animal or human cell. The mesenchymal cells of the disclosure can be removed (isolated) from a tissue and may be cultured to increase the population size of the cells. The animal or human cells may be maintained in a viable state either by culturing by serial passage in a culture medium or cryopreserved by methods well-known in the art. The cells may be obtained from the animal or human individual that has received an injury desired to be repaired by administration of the engineered cells of the disclosure or from a different individual.

Cells, and their extracellular vesicle such as an exosomes that are contemplated for use in the methods of the present disclosure may be derived from the same subject to be treated (autologous to the subject) or they may be derived from a different subject, preferably of the same species, (allogeneic to the subject).

Commercially available media may be used for the growth, culture and maintenance of mesenchymal stem cells. Such media include but are not limited to Dulbecco's modified Eagle's medium (DMEM). Components in such media that are useful for the growth, culture and maintenance of mesenchymal stem cells include but are not limited to amino acids, vitamins, a carbon source (natural and non-natural), salts, sugars, plant derived hydrolysates, sodium pyruvate, surfactants, ammonia, lipids, hormones or growth factors, buffers, non-natural amino acids, sugar precursors, indicators, nucleosides and/or nucleotides, butyrate or organics, DMSO, animal derived products, gene inducers, non-natural sugars, regulators of intracellular pH, betaine or osmoprotectant, trace elements, minerals, non-natural vitamins. Additional components that can be used to supplement a commercially available tissue culture medium include, for example, animal serum (e.g., fetal bovine serum (FBS), fetal calf serum (FCS), horse serum (HS)), antibiotics (e.g., including but not limited to, penicillin, streptomycin, neomycin sulfate, amphotericin B, blasticidin, chloramphenicol, amoxicillin, bacitracin, bleomycin, cephalosporin, chlortetracycline, zeocin, and puromycin), and glutamine (e.g., L-glutamine). Mesenchymal stem cell survival and growth also depends on the maintenance of an appropriate aerobic environment, pH, and temperature. Mesenchymal stem cells can be maintained using methods known in the art (see, for example, Pittenger et al., (1999) Science 284:143-147).

The term "cell therapy" as used herein refers to the introduction of new cells into a tissue in order to treat a disease and represents a method for repairing or replacing diseased tissue with healthy tissue.

The term "clonal" refers to a cell or a group of cells that have arisen from a single cell through numerous cycles of cell division. The cells of a clonal population are genetically identical. A clonal population can be a heterogeneous population such that the cells can express a different set of genes at a specific point in time.

The term "composition" as used herein refers to a product comprising the specified ingredients in the specified amounts, as well as any product which results, directly or indirectly, from combination of the specified ingredients in the specified amounts. Such a term in relation to a pharmaceutical composition is intended to encompass a product comprising the active ingredient(s), and the inert ingredient(s) that make up the carrier, as well as any product which results, directly or indirectly, from combination, complexation, or aggregation of any two or more of the ingredients, or from dissociation of one or more of the ingredients, or from other types of reactions or interactions of one or more of the ingredients. Accordingly, the pharmaceutical compositions of the present disclosure encompass any composition made by admixing a compound of the present disclosure and a pharmaceutically acceptable carrier.

When a compound of the present disclosure is used contemporaneously with one or more other drugs, a pharmaceutical composition containing such other drugs in addition to the compound of the present disclosure is contemplated. Accordingly, the pharmaceutical compositions of the present disclosure include those that also contain one or more other active ingredients, in addition to a compound of the present disclosure. The weight ratio of the compound of the present disclosure to the second active ingredient may be varied and will depend upon the effective dose of each ingredient. Generally, an effective dose of each will be used. Thus, for example, but not intended to be limiting, when a compound of the present disclosure is combined with another agent, the weight ratio of the compound of the present disclosure to the other agent will generally range from about 1000:1 to about 1:1000, preferably about 200:1 to about 1:200. Combinations of a compound of the present disclosure and other active ingredients will generally also be within the aforementioned range, but in each case, an effective dose of each active ingredient should be used. In such combinations the compound of the present disclosure and other active agents may be administered separately or in conjunction. In addition, the administration of one element may be prior to, concurrent to, or subsequent to the administration of other agent(s).

The term "culturing" as used herein refers to growing cells or tissue under controlled conditions suitable for survival, generally outside the body (e.g., *ex vivo* or *in vitro*). The term includes "expanding," "passaging," "maintaining," etc. when referring to cell culture of the process of culturing. Culturing cells can result in cell growth, differentiation, and/or division.

The term "derived from" as used herein refers to cells or a biological sample (e.g., blood, tissue, bodily fluids, etc.) and indicates that the cells or the biological sample were obtained from the stated source at some point in time. For example, a cell derived from an individual can represent a primary cell obtained directly from the individual (i.e., unmodified). In some instances, a cell derived from a given source undergoes one or more rounds of cell division and/or cell differentiation such that the original cell no longer exists, but the continuing cell (e.g., daughter cells from all generations) will be understood to be derived from the same source. The term includes directly obtained from, isolated and cultured, or obtained, frozen, and thawed. The term "derived from" may also refer to a component or fragment of a cell obtained from a tissue or cell, including, but not limited to, a protein, a nucleic acid, a membrane or fragment of a membrane, and the like.

The term "disaggregating" includes separating, dislodging, or dissociating cells or tissue using mechanical or enzymatic disruption to isolate single cells or small clusters of cells. In some instances, enzymatic disruption can be replaced with one of more enzyme alternatives having substantially the same effect as the enzyme.

The term "effective amount" as used herein refers to the amount of a therapy which is sufficient to reduce or ameliorate the severity and/or duration of a disorder or one or more symptoms thereof, inhibit or prevent the advancement of a disorder, cause regression of a disorder, inhibit or prevent the recurrence, development, onset or progression of one or more symptoms associated with a disorder, detect a disorder, or enhance or improve the prophylactic or therapeutic effect(s) of another therapy (e.g., prophylactic or therapeutic agent).

The term "endothelial cell" refers to a cell necessary for the formation and development of new blood vessel from pre-existing vessels (e.g., angiogenesis). Typically, endothelial cells are the thin layer of cells that line the interior surface of blood vessels and lymphatic vessels. Endothelial cells are involved in various aspects of vascular biology, including atherosclerosis, blood clotting, inflammation, angiogenesis, and control of blood pressure.

The term "exosomes" as used herein refers to small secreted vesicles (typically about 30 nm to about 150 nm(or largest dimension where the particle is not spheroid)) that may contain, or have present in their membrane, nucleic acid, protein, or other biomolecules and may serve as carriers of this cargo between diverse locations in a body or biological system. The term "exosomes" as used herein advantageously refers to extracellular vesicles that can have therapeutic properties, including, but not limited to stem cell exosomes such as cardiac stem cell or mesenchymal stem cells.

Exosomes may be isolated from a variety of biological sources including mammals such as mice, rats, guinea pigs, rabbits, dogs, cats, bovine, horses, goats, sheep, primates or humans. Exosomes can be isolated from biological fluids such as serum, plasma, whole blood, urine, saliva, breast milk, tears, sweat, joint fluid, cerebrospinal fluid, semen, vaginal fluid, ascetic fluid and amniotic fluid. Exosomes may also be isolated from experimental samples such as media taken from cultured cells ("conditioned media", cell media, and cell culture media).

Exosomes may also be isolated from tissue samples such as surgical samples, biopsy samples, and cultured cells. When isolating exosomes from tissue sources it may be necessary to homogenize the tissue in order to obtain a single cell suspension followed by lysis of the cells to release the exosomes. When isolating exosomes from tissue samples it is important to select homogenization and lysis procedures that do not result in disruption of the exosomes.

Exosomes may be isolated from freshly collected samples or from samples that have been stored frozen or refrigerated. Although not necessary, higher purity exosomes may be obtained if fluid samples are clarified before precipitation with a volume-excluding polymer, to remove any debris from the sample. Methods of clarification include centrifugation, ultracentrifugation, filtration or ultrafiltration.

The genetic information within the extracellular vesicle such as an exosome may easily be transmitted by fusing to the membranes of recipient cells, and releasing the genetic information into the cell intracellularly. Though exosomes as a general class of compounds represent great therapeutic potential, the general population of exosomes are a combination of several class of nucleic acids and proteins which have a constellation of biologic effects both advantageous and deleterious. In fact, there are over 1000 different types of exosomes.

The term "extracellular vesicle" as used herein can refers to a membrane vesicle secreted by cells that may have a larger diameter than that referred to as an "exosome". Extracellular vesicles (alternatively named "microvesicle" or "membrane vesicle") may have a diameter (or largest dimension where the particle is not spheroid) of between about 10 nm to about 5000 nm (e.g., between about 50 nm and 1500 nm, between about 75 nm and 1500 nm, between about 75 nm and 1250 nm, between about 50 nm and 1250 nm, between about 30 nm and 1000 nm, between about 50 nm and 1000 nm, between about 100 nm and 1000 nm, between about 50 nm and 750 nm, etc.). Typically, at least part of the membrane of the extracellular vesicle is directly obtained from a cell (also known as a donor cell). Extracellular vesicles suitable for use in the compositions and methods of the present disclosure may originate from cells by membrane inversion, exocytosis, shedding, blebbing, and/or budding. Extracellular vesicles may originate from the same population of donor cells yet different subpopulations of extracellular vesicles may exhibit different surface/lipid characteristics. Alternative names for extracellular vesicles include, but are not limited to, exosomes, ectosomes, membrane particles, exosome-like particles, and apoptotic vesicles. Depending on the manner of generation (e.g., membrane inversion, exocytosis, shedding, or budding), the extracellular vesicles contemplated herein may exhibit different surface/lipid characteristics.

The term "formulation" as used herein refers to a composition that may be a stock solution of the components, or a composition, preferably including a dilutant such as water or other pharmaceutically acceptable carrier that may be available for distribution including to a patient or physician.

The disclosure provides dosage forms, formulations, and methods that provide advantages and/or beneficial pharmacokinetic profiles, more particularly sustained pharmacokinetic profiles. A composition of the disclosure can be utilized in dosage forms in pure or substantially pure form, in the form of its pharmaceutically acceptable salts, and also in other forms including anhydrous or hydrated forms.

A beneficial pharmacokinetic profile may be obtained by administering a formulation or dosage form suitable for once, twice a day, or three times a day, or more administration comprising one or more composition of the disclosure present in an amount sufficient to provide the required concentration or dose of the composition to an environment of use to treat a disease disclosed herein, in particular a cancer.

Embodiments of the disclosure relate to a dosage form comprising one or more composition of the disclosure that can provide peak plasma concentrations of the composition of between about 0.001 to 2 mg/ml, 0001 to 1 mg/ml, 0.0002 to 2 mg/ml, 0.005 to 2 mg/ml, 001 to 2 mg/ml, 0.05 to 2 mg/ml, 0.001 to 0.5 mg/ml, 0.002 to 1 mg/ml, 0.005 to 1 mg/ml, 0.01 to 1 mg/ml, 005 to 1 mg/ml, or 0.1 to 1 mg/ml. The disclosure also provides a formulation or dosage form comprising one or more composition of the disclosure that provides an elimination t_{1/2} of 0.5 to 20 h, 0.5 to 15 h, 0.5 to 10 h, 0.5 to 6 h, 1 to 20 h, 1 to 15 h, 1 to 10 h, or 1 to 6 h.

A subject may be treated with a composition of the disclosure or composition or unit dosage thereof on substantially any desired schedule. They may be administered one or more times per day, in particular 1 or 2 times per day, once per week, once a month or continuously. However, a subject may be treated less frequently, such as every other day or once a week, or more frequently. A composition or composition may be administered to a subject for about or at least about 24 hours, 2 days, 3 days, 1 week, 2 weeks to 4 weeks, 2 weeks to 6 weeks, 2 weeks to 8 weeks, 2 weeks to 10 weeks, 2 weeks to 12 weeks, 2 weeks to 14 weeks, 2 weeks to 16 weeks, 2 weeks to 6 months, 2 weeks to 12 months, 2 weeks to 18 months, 2 weeks to 24 months, or for more than 24 months, periodically or continuously.

A beneficial pharmacokinetic profile can be obtained by the administration of a formulation or dosage form suitable for once, twice, or three times a day administration, preferably twice a day administration comprising one or more composition of the disclosure present in an amount sufficient to provide the requited dose of the composition. The required dose of a composition of the disclosure administered once twice, three times or more daily is about 0.01 to 3000 mg/kg, 0.01 to 2000 mg/kg, 0.5 to 2000 mg/kg, about 0.5 to 1000 mg/kg, 0.1 to 1000 mg/kg, 0.1 to 500 mg/kg, 0.1 to 400 mg/kg, 0.1 to 300 mg/kg, 0.1 to 200 mg/kg, 0.1 to 100 mg/kg, 0.1 to 50 mg/kg, 0.1 to 20 mg/kg, 0.1 to 10 mg/kg, 0.1 to 6 mg/kg, 0.1 to 5 mg/kg, 0.1 to 3 mg/kg, 0.1 to 2 mg/kg, 0.1 to 1 mg/kg, 1 to 1000 mg/kg, 1 to 500 mg/kg, 1 to 400 mg/kg, 1 to 300 mg/kg, 1 to 200 mg/kg, 1 to 100 mg/kg, 1 to 50 mg/kg, 1 to 20 mg/kg, 1 to 10 mg/kg, 1 to 6 mg/kg, 1 to 5 mg/kg, or 1 to 3 mg/kg, or 1 to 2.5 mg/kg, or less than or about 10 mg/kg, 5 mg/kg, 2.5 mg/kg, 1 mg/kg, or 0.5 mg/kg twice daily or less Certain dosage forms and formulations may minimize the variation between peak and trough plasma and/or brain levels of compositions of the disclosure and in particular provide a sustained therapeutically effective amount of the compositions.

The disclosure also contemplates a formulation or dosage form comprising amounts of one or more composition of the disclosure that results in therapeutically effective amounts of the composition over a dosing period, in particular a 24 h dosing period. The therapeutically effective amounts of a composition of the disclosure are between about 0.1 to 1000 mg/kg, 0.1 to 500 mg/kg, 0.1 to 400 mg/kg, 0.1 to 300 mg/kg, 0.1 to 200 mg/kg, 0.1 to 100 mg/kg, 0.1 to 75 mg/kg, 0.1 to 50 mg/kg, 0.1 to 25 mg/kg, 0.1 to 20 mg/kg, 0.1 to 15 mg/kg, 0.1 to 10 mg/kg, 0.1 to 9 mg/kg, 0.1 to 8 mg/kg, 0.1 to 7 mg/kg, 0.1 to 6 mg/kg, 0.1 to 5 mg/kg, 0.1 to 4 mg/kg, 0.1 to 3 mg/kg, 0.1 to 2 mg/kg, or 0.1 to 1 mg/kg.

A medicament or treatment of the disclosure may comprise a unit dosage of at least one composition of the disclosure to provide therapeutic effects. A "unit dosage" or "dosage unit" refers to a unitary, i.e. a single dose, which is capable of being administered to a patient, and which may be readily handled and packed, remaining as a physically and chemically stable unit dose comprising either the active agents as such or a mixture with one or more solid or liquid pharmaceutical excipients, carriers, or vehicles.

The terms "generate", "generation", and "generating" as used herein shall be given their ordinary meaning and shall refer to the production of new cells in a subject and optionally the further differentiation into mature, functioning cells. Generation of cells may comprise regeneration of the cells. Generation of cells comprises improving survival, engraftment and/or proliferation of the cells.

The term "growth factors" as used herein refers to proteins, peptides or other molecules having a growth, proliferative, differentiation, or trophic effect on cells. Such factors may be used for inducing proliferation or differentiation and can include, for example, any trophic factor that allows cells to proliferate, including any molecule which binds to a receptor on the surface of the cell to exert a trophic, or growth-inducing effect on the cell. Such factors include paracrine factors secreted by stem cells and which may induce or sustain proliferation or differentiation by cells in close proximity to the biomimetic microparticles of the disclosure. Factors include, but are not limited to, Adrenomedullin (AM); Angiopoietin (Ang); Autocrine motility factor; Bone morphogenetic proteins (BMPs); Ciliary neurotrophic factor family; Ciliary neurotrophic factor (CNTF); Leukemia inhibitory factor (LIF); Interleukin-6 (IL-6); Colony-stimulating factors; Macrophage colony-stimulating factor (m-CSF); Granulocyte colony-stimulating factor (G-CSF); Granulocyte macrophage colony-stimulating factor (GM-CSF); Epidermal growth factor (EGF); Ephrin A1; Ephrin A2; Ephrin A3; Ephrin A4; Ephrin A5; Ephrin B1; Ephrin B2; Ephrin B3; Erythropoietin (EPO); Fibroblast growth factor (FGF); Foetal Bovine Somatotrophin (FBS); GDNF family of ligands: Glial cell line-derived neurotrophic factor (GDNF); Neurturin; Persephin; Artemin; Growth differentiation factor-9 (GDF9); Hepatocyte growth factor (HGF); Hepatoma-derived growth factor (HDGF); Insulin; Insulin-like growth factor-1 (IGF-1); Insulin-like growth factor-2 (IGF-2); Interleukins: IL-1; IL-2; IL-3; IL-4; IL-5; IL-6; IL-7; Keratinocyte growth factor (KGF); Migration-stimulating factor (MSF); Macrophage-stimulating protein (MSP); Myostatin (GDF-8); Neuregulins; Neuregulin 1 (NRG1); Neuregulin 2 (NRG2); Neuregulin 3 (NRG3); Neuregulin 4 (NRG4); Brain-derived neurotrophic factor (BDNF); Nerve growth factor (NGF); Neurotrophin-3 (NT-3); Neurotrophin-4 (NT-4); Placental growth factor (PGF); Platelet-derived growth factor (PDGF); Renalase (RNLS); T-cell growth factor (TCGF); Thrombopoietin (TPO); Transforming growth factors; Transforming growth factor alpha (TGF-α); Transforming growth factor beta (TGF-β); Tumor necrosis factor-alpha (TNF-α); Vascular endothelial growth factor (VEGF), and the like.

The term "isolating" or "isolated" when referring to a cell or a molecule (e.g., nucleic acids or protein) indicates that the cell or molecule is or has been separated from its natural, original or previous environment. For example, an isolated cell can be removed from a tissue derived from its host individual, but can exist in the presence of other cells (e.g., in culture), or be reintroduced into its host individual.

The terms "lung progenitor cells" and "lung stem cells" as used herein can refer to a population of progenitor cells derived from human or animal lung tissue.

The term "mesenchymal stem cells (MSCs)" as used herein refers to progenitor cells having the capacity to differentiate into neuronal cells, adipocytes, chondrocytes, osteoblasts, myocytes, cardiac tissue, and other endothelial and epithelial cells. (See for example Wang, (2004) Stem Cells 22: 1330-1337; McElreavey (1991) Biochem. Soc. Trans. 1: 29s; Takechi (1993) Placenta 14: 235-245; Yen (2005) Stem Cells; 23: 3-9). These cells have been characterized to express, and thus be positive for, one or more of CD13, CD29, CD44, CD49a, b, c, e, f, CD51, CD54, CD58, CD71, CD73, CD90, CD102, CD105, CD106, CDw119, CD120a, CD120b, CD123, CD124, CD126, CD127, CD140a, CD166, P75, TGF-□IR, TGF-□IIR, HLA-A, B, C, SSEA-3, SSEA-4, D7 and PD-L1.

Mesenchymal stem cells may be harvested from a number of sources including, but not limited to, bone marrow, blood, periosteum, dermis, umbilical cord blood and/or matrix (e.g., Wharton's Jelly), and placenta. Example of methods for obtaining mesenchymal stem cells reference can be found, for example, in U.S. Pat. No. 5,486,359.

The terms "modulating the proliferative status" or "modulating the proliferation" as used herein refers to the ability of a compound to alter the proliferation rate of a population of stem or progenitor cells. A compound may be toxic, wherein the proliferation of the cells is slowed or halted, or the proliferation may be enhanced such as, for example, by the addition to the cells of a cytokine or growth factor.

The term "multipotent" refers to a cell having the potential to differentiate into multiple, yet a limited number of cell types or cell lineages. Typically, these cells are considered unspecialized cells that have the ability to self-renew and become specialized cells with specific functions and characteristics.

The term "paracrine signaling" as used herein refers to a form of cell-cell communication in which a cell produces a signal to induce changes in nearby cells, altering the behavior or differentiation of those cells. Signaling molecules known as paracrine factors diffuse over a relatively short distance (local action), as opposed to endocrine factors (hormones which travel considerably longer distances via the circulatory system), juxtacrine interactions, and autocrine signaling. Cells that produce paracrine factors secrete them into the immediate extracellular environment. Factors then travel to nearby cells in which the gradient of factor received determines the outcome

Although paracrine signaling elicits a diverse array of responses in the induced cells, most paracrine factors utilize a relatively narrow set of receptors and pathways. Different organs in the body, even between different species, can utilize similar sets of paracrine factors in differential development. Highly conserved receptors and pathways can be organized into four major families based on similar structures: Fibroblast growth factor (FGF) family, Hedgehog family, Wnt family, and TGF-β superfamily. Binding of a paracrine factor to its respective receptor initiates signal transduction cascades, eliciting different responses.

For paracrine factors to induce a response in the receiving cell, that cell must have the appropriate receptors available on the cell membrane to receive the signals, also known as being competent. Additionally, the responding cell must also have the ability to be mechanistically induced.

The term "pharmaceutical composition" as used herein refers to a composition that can be administered to an animal or human patient to treat or prevent a disease or pathological condition in the patient. The compositions can be formulated according to known methods for preparing pharmaceutically useful compositions. Furthermore, as used herein, the phrase "pharmaceutically acceptable carrier" means any of the standard pharmaceutically acceptable carriers. The pharmaceutically acceptable carrier can include diluents, adjuvants, and vehicles, as well as implant carriers, and inert, non-toxic solid or liquid fillers, diluents, or encapsulating material that does not react with the active ingredients of the invention. Examples include, but are not limited to, phosphate buffered saline, physiological saline, water, and emulsions, such as oil/water emulsions. The carrier can be a solvent or dispersing medium containing, for example, ethanol, polyol (for example, glycerol, propylene glycol, liquid polyethylene glycol, and the like), suitable mixtures thereof, and vegetable oils. Formulations containing pharmaceutically acceptable carriers are described in a number of sources which are well known and readily available to those skilled in the art. For example, Remington's Pharmaceutical Sciences (Martin E W, Remington's Pharmaceutical Sciences, Easton Pa., Mack Publishing Company, 19.sup.th ed., 1995) describes formulations that can be used in connection with the subject invention. Formulations suitable for parenteral administration include, for example, aqueous sterile injection solutions, which may contain antioxidants, buffers, bacteriostats, and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze dried (lyophilized) condition requiring only the condition of the sterile liquid carrier, for example, water for injections, prior to use. Extemporaneous injection solutions and suspensions may be prepared from sterile powder, granules, tablets, etc. It should be understood that in addition to the ingredients particularly mentioned above, the formulations of the subject invention can include other agents conventional in the art having regard to the type of formulation in question.

The term "pharmaceutically acceptable carrier, excipient, or vehicle" as used herein refers to a medium which does not interfere with the effectiveness or activity of an active ingredient and which is not toxic to the hosts to which it is administered and which is approved by a regulatory agency of the Federal or a state government or listed in the U.S. Pharmacopeia or other generally recognized pharmacopeia for use in animals, and more particularly in humans. A carrier, excipient, or vehicle includes diluents, binders, adhesives, lubricants, disintegrates, bulking agents, wetting or emulsifying agents, pH buffering agents, and miscellaneous materials such as absorbents that may be needed in order to prepare a particular composition. Examples of carriers etc. include but are not limited to saline, buffered saline, dextrose, water, glycerol, ethanol, and combinations thereof. The use of such media and agents for an active substance is well known in the art.

The terms "pharmaceutically acceptable" or "pharmacologically acceptable" as used herein refer to a material that is not biologically or otherwise undesirable, i.e., the material may be administered to an individual in a formulation or composition without causing any undesirable biological effects or interacting in a deleterious manner with any of the components of the composition in which it is contained.

The term "progenitor cell" as used herein refers to a cell that has the capacity to differentiate into a specific type of cell, as well as replicate to generate a daughter cell substantially equivalent to itself. In some instances, a progenitor cell undergoes limited self-renewal such that it does not self-replicate indefinitely.

The term "proliferative status" as used herein refers to whether a population of cells including, but not limited to, mesenchymal stem or progenitor cells, or a subpopulation thereof, are dividing and thereby increasing in number, in the quiescent state, or whether the cells are not proliferating, dying or undergoing apoptosis.

The term "pulmonary fibrosis" refers to pathological fibrosis of the lungs whether arising from disease or injury. The term encompasses IPF, pleural fibrosis, subpleural fibrosis, pulmonary fibrosis, Usual Interstitial Pneumonia (UIP) and drug-induced lung fibroses.

The terms "regenerate," "regeneration" and "regenerating" as used herein refer to the process of growing and/or developing new tissue to replace tissue that has been injured, for example, by viral infection, chemically-induced injury, or other disease. Tissue regeneration may comprise activation and/or enhancement of cell proliferation.

The term "secretome" as used herein refers to polypeptides secreted into and collected from an extracellular culture medium and include growth factors, chemokines, cytokines, adhesion molecules, proteases and shed receptors. Human protein-coding genes (39%, 19613 genes) are predicted to have either a signal peptide and/or at least one transmembrane region suggesting active transport of the corresponding protein out of the cell (secretion) or location in one of the numerous membrane systems in the cell. Non-protein components, such as lipid, micro-RNAs and messenger-RNA can also be secreted by cells via both microvesicles (100 to more than1000 nm diameter) shed from the plasma membrane and exosomes (from about 30 nm to about 150 nm diameter) that are released via endosomal-exocytosis event. Factors present in both these organelles accounts for up to 42% of the secretome and are included in the collective secretome. Accordingly, the conditioned cell culture media of the present disclosure may include some or all secreted proteins, nucleic acids, microvesicles, and exosomes generated from a population of cells, including but not limited to, or isolated examples of such.

The term "self-renewal" or "self-renewing" as used herein refers to the ability of a cell to divide through numerous cycles of cell division and generate a daughter with the same characteristics as the parent cell. The other daughter cell can have characteristics different from its parent cell. The term includes the ability of a cell to generate an identical genetic copy of itself (e.g., clone) by cell division. For example, a self-renewing cardiac progenitor cell can divide to form one daughter cardiac progenitor cell and another daughter cell committed to differentiation to a cardiac lineage such as an endothelial, smooth muscle or cardiomyocyte cell. In some instances, a self-renewing cell does not undergo cell division forever.

The term "stem cell" as used herein refers to cells that have the capacity to self-renew and to generate differentiated progeny. The term "pluripotent stem cells" refers to stem cells that have complete differentiation versatility, i.e., the capacity to grow into any of the fetal or adult mammalian body's approximately 260 cell types. For example, pluripotent stem cells have the potential to differentiate into three germ layers: endoderm (e.g., blood vessels), mesoderm (e.g., muscle, bone and blood) and ectoderm (e.g., epidermal tissues and nervous system), and therefore, can give rise to any fetal or adult cell type.

The terms "subject" and "patient" as used herein include humans, mammals (*e.g*., cats, dogs, horses, *etc*.), living cells, and other living organisms. A living organism can be as simple as, for example, a single eukaryotic cell or as complex as a mammal. Typical hosts to which embodiments of the present disclosure may be administered will be mammals, particularly primates, especially humans. For veterinary applications, a wide variety of subjects will be suitable, *e.g*., livestock such as cattle, sheep, goats, cows, swine, and the like; poultry such as chickens, ducks, geese, turkeys, and the like; and domesticated animals particularly pets such as dogs and cats. For diagnostic or research applications, a wide variety of mammals will be suitable subjects, including rodents (*e.g*., mice, rats, hamsters), rabbits, primates, and swine such as inbred pigs and the like. In some embodiments, a system includes a sample and a subject. The term "living host" refers to host or organisms noted above that are alive and are not dead. The term "living host" refers to the entire host or organism and not just a part excised (*e.g*., a liver or other organ) from the living host.

The term "therapeutic effect" as used herein refers to an effect of a composition of the disclosure, in particular a formulation or dosage form, or method disclosed herein. A therapeutic effect may be a sustained therapeutic effect that correlates with a continuous concentration of a compound of the disclosure over a dosing period, in particular a sustained dosing period. A therapeutic effect may be a statistically significant effect in terms of statistical analysis of an effect of a compound of the disclosure versus the effects without the compound.

The term "therapeutically effective amount" as used herein to the amount or dose of an active compound of the disclosure or composition comprising the same, that will lead to one or more desired effects, in particular, one or more therapeutic effects or beneficial pharmacokinetic profiles. A therapeutically effective amount of a substance can vary according to factors such as the disease state, age, sex, and weight of the subject, and the ability of the substance to elicit a desired response in the subject. A dosage regimen may be adjusted to provide the optimum therapeutic response or pharmacokinetic profile. For example, several divided doses may be administered daily or the dose may be proportionally reduced as indicated by the exigencies of the therapeutic situation.

The term "tissue injury" as used herein refers to damage to a lung tissue of an animal or human.

The terms "treatment," "therapy," "amelioration" and the like refer to any reduction in the severity of symptoms. As used herein, the terms "treat" and "prevent" are not intended to be absolute terms. Treatment can refer to any delay in onset, amelioration of symptoms, improvement in patient survival, repair/regeneration of heart tissue or blood vessels, increase in survival time or rate, etc. The effect of treatment can be compared to an individual or pool of individuals not receiving the treatment. In some instances, the effect can be the same patient prior to treatment or at a different time during the course of therapy. In some aspects, the severity of disease, disorder or injury is reduced by at least 10%, as compared, e.g., to the individual before administration or to a control individual (e.g., healthy individual or an individual no longer having the disease, disorder or injury) not undergoing treatment. In some instances, the severity of disease, disorder or injury is reduced by at least 20%, 25%, 50%, 75%, 80%, or 90%. In some cases, the symptoms or severity of disease are no longer detectable using standard diagnostic techniques.

### Description

Aspects of the disclosure relate to methods of treating pulmonary lung disease, and in particular pulmonary fibrosis. The methods comprises administering to a patient a pharmaceutical composition that comprises lung spheroid cell-conditioned culture medium that may comprise at least one cell secreted factor such as a peptide, protein, or nucleic acid or lung spheroid cell-derived exosomes as described herein, and in an amount sufficient to reduce significantly one or more of the following pathological conditions: lung edema, a lung pathology associated with lung fibrosis and hydroxyproline accumulation or fibrogenic proteins such as collagen and elastin.

It has been shown that therapeutic adult lung cells termed lung spheroid cells (LSCs), contains a heterogeneous population of cells expressing lung epithelial (Epcam, AQP5, ProSPC and CCSP)- and mesenchymal (CD90 and CD105)-specific markers. The purpose of the present study was to examine the therapeutic effects of LSC-derived conditioned media in pulmonary fibrosis. It has been reported that mesenchymal stem cell (MSCs) secretome or conditioned media indeed reproduces the therapeutic effects seen in MSC cell therapy in some diseases, such as osteoarthritis, bronchopulmonary dysplasia, and multiple sclerosis. These studies include MSC-derived CM as a comparator treatment to test for non-inferiority show that lung spheroid cell-conditioned media (LSC-CM) promotes lung repair in pulmonary fibrosis, in a fashion that is superior to its MSC counterpart.

Idiopathic pulmonary fibrosis (IPF) is a fatal form of idiopathic interstitial lung disease in which persistent lung injuries result in scar tissue formation. As fibrosis thickens, the lung tissue becomes stiff and losses the ability to facilitate gas exchange and provide cells with the oxygen it needs. Currently, IPF has few treatment options and no effective therapies aside from a lung transplant. The embodiments of the methods of the disclosure using lung spheroid cell-derived conditioned media (LSC-CM) and lung spheroid cell exosomes (LSC-EXO) to treat different models of lung injury are presented.

Various animal models have been developed to mimic the pathological hallmarks of human IPF. Due to the unknown cause of the disease, a true IPF animal model does not exist. The Bleo model of induced pulmonary fibrosis is widely used for the study of IPF and is arguably the most clinically relevant. To support any therapeutic effects found in the Bleo model, a silica-induced pulmonary fibrosis model was also tested.

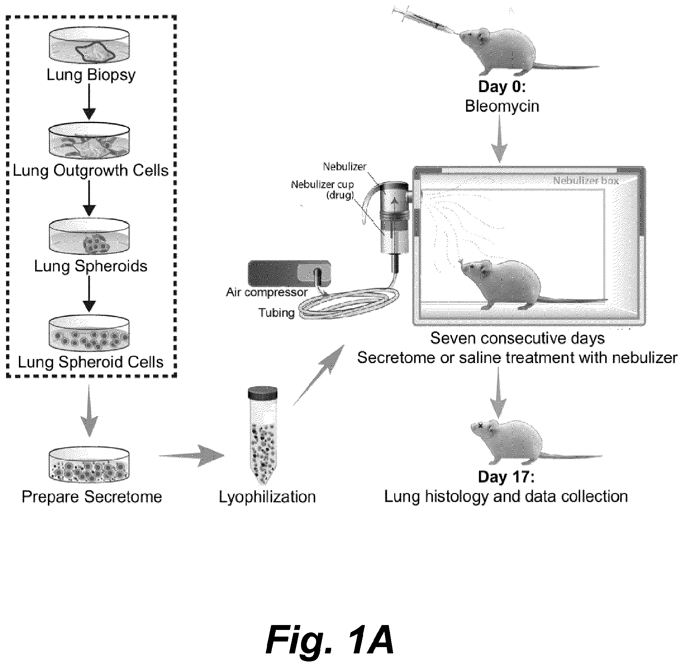

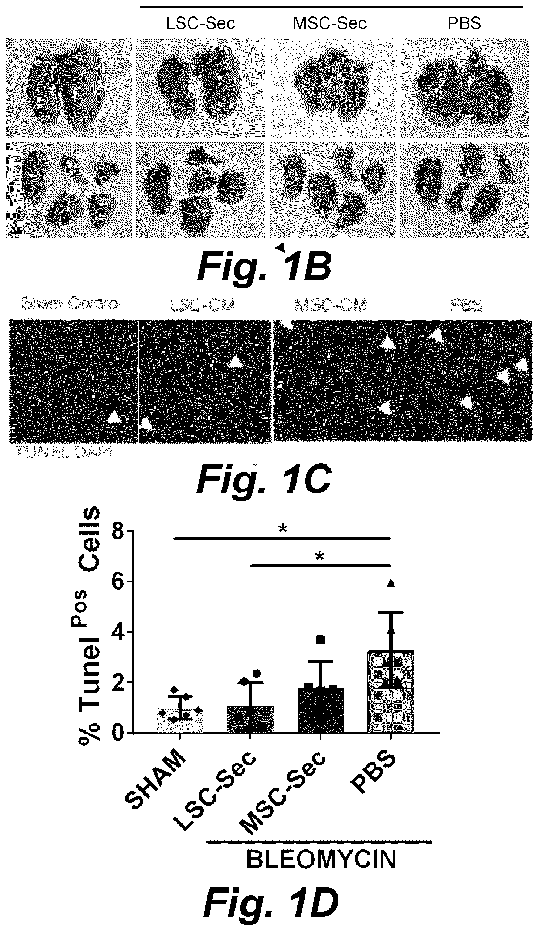

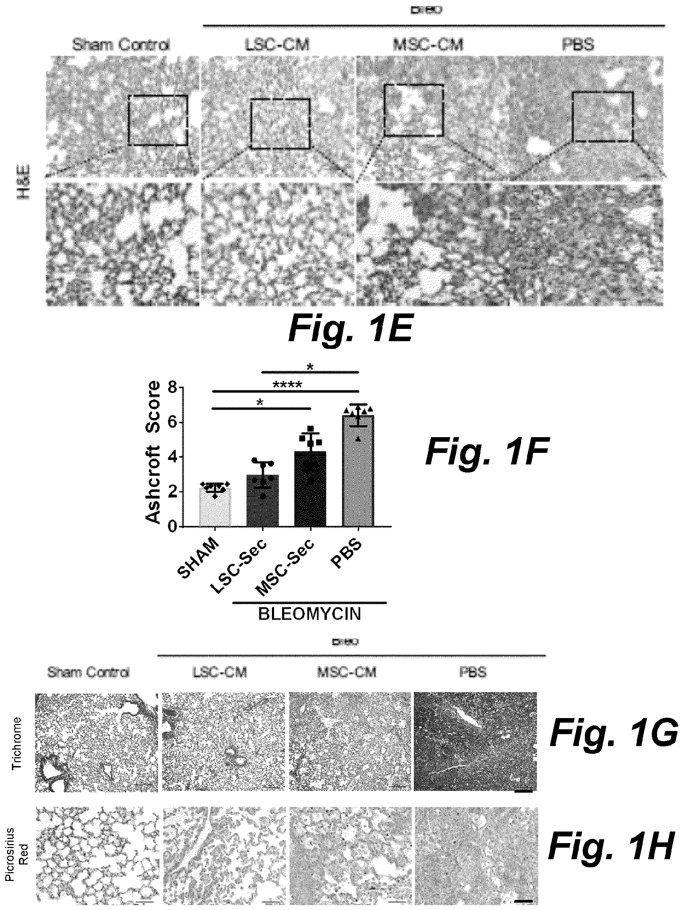

Both the Bleo and silica mouse models presented significant alveolar epithelial damage and ECM deposition (Figs. 1E-1I, 3C-3G, and 5E-5H). Shown in the rat model was a significant decline in pulmonary function following Bleo injury (Figs. 6C-6I). It has been demonstrated that cell-free CM derived from stem cells can achieve similar or superior protective and regenerative abilities as compared to the cells themselves itself. The latest observations show that LSC-CM was capable of reversing fibrosis caused by Bleo, and also that caused by silica particles (Figs. 1-3). However, the regenerative effects of LSC-CM were more robust in the Bleo model than in the silica model. LSC-CM inhalation treatment in the Bleo-instilled mice was able to rescue damaged lungs back to similar levels as those of healthy sham control in terms of decreased apoptotic cells, fibrotic score, hydroxyproline content, and significant recovery of AT1 and AT2 cell populations (Figs. 1C-1I and 2A-2D).

The effects of CM treatment in the silica model, while reversing fibrosis compared to the non-treated controls (PBS treated), did not reverse the damage back to levels as seen in healthy sham controls. This could be due to the difference in the animal models. Silica, unlike Bleo induced injury, is caused by the physical presence of the particles depositing into the lung, causing formation of fibrotic nodules. Histological examination showed a decrease in fibrotic tissues surrounding the nodules in CM treated groups as compared to the PBS control, but CM therapy was not enough to resolve the nodules themselves (Figs. 3C, 3E, and 3F). It is possible that seven days of treatment was not sufficient to reverse the damage caused by the silica particles. For certain types of lung injury where particulate matter is retained in the lungs causing dysfunction, such as silicosis it longer continuous CM treatment can be required to not only resolve the fibrosis, but also clear the particles.

The field of regenerative medicine utilizes many different types of stem cells for research and clinical applications, but mesenchymal stem cells remain the most widely used, partly due to their immunomodulating abilities and ease of isolation. Therefore, the regenerative benefits of LSC-CM against MSC-CM were compared. Thus, in both Bleo and silica models it was demonstrated that while MSC-CM inhalation therapy had regenerative activities in treating pulmonary fibrosis, LSC-CM was superior to MSC-CM in all measures (Figs. 1-3).

The molecular mechanism(s) underlying CM-mediated lung repair through proteomic analyses were elucidated. Such analyses revealed downregulation of αSMA (a myofibroblast marker) and pro-inflammatory/pro-fibrotic cytokines, possibly through the upregulation of MMP2 activity (Figs. 2E-2G). It has been shown that there is a diverse role of MMP activities in PF, suggesting that in the early stages of Bleo-induced PF MMPs play a role in disease pathogenesis. However, MMP activity in the late stage may play a role in the repair process.

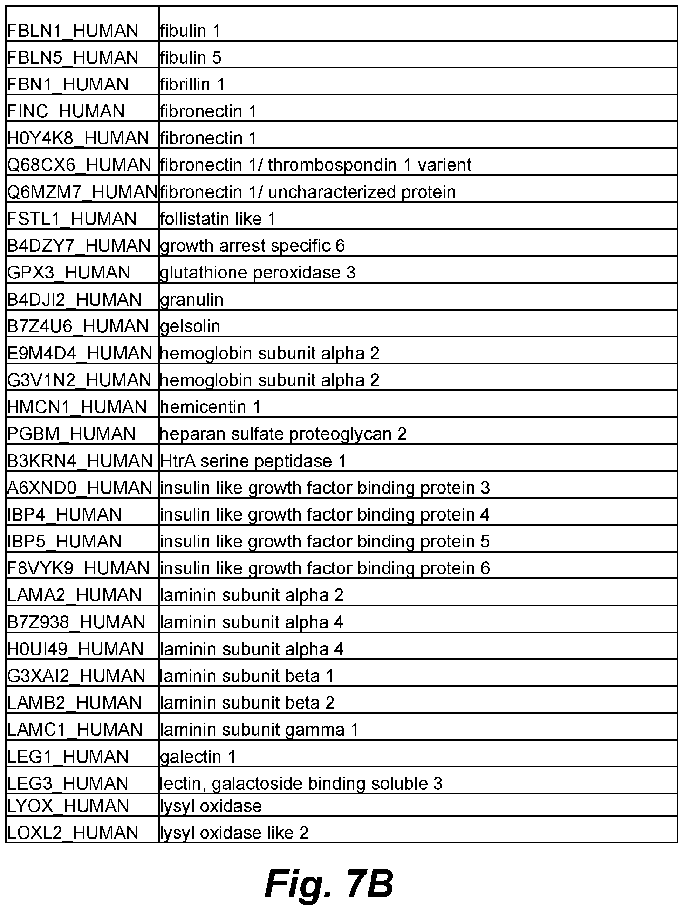

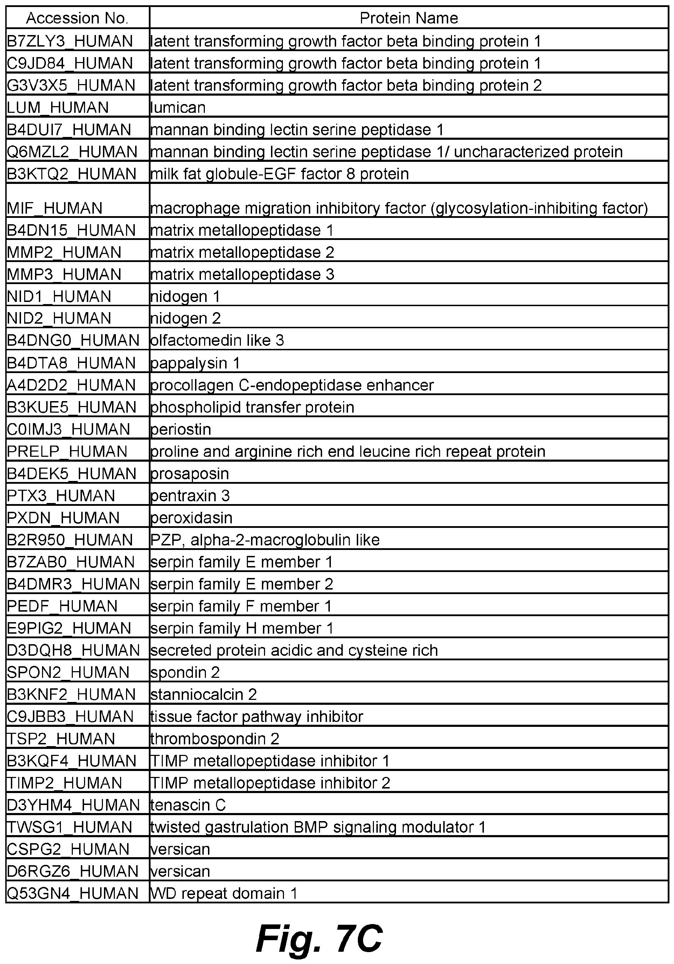

Mass spectrometry analysis of the LSC secretome revealed proteins related to the complement system (C1r and decorin) as well as Wnt signaling pathway (DKK3) (Fig. 4, Fig. 7). Additionally, an abundance of ECM remodeling, pro-angiogenic, and cell proliferation proteins was also identified, suggesting that LSC-CM contains proteins able to not only break down and reverse the fibrosis already in place, but also promote epithelial and vascular repair.

Conditioned media does not merely contain soluble proteins, but also insoluble substances, such as exosomes. Exosomes are an emerging field of biomedical research that has seen an explosion of discoveries and interest in the last two decades. Therapeutic effects of stem cells have been attributed to the miRNAs found in the secreted exosomes. Therefore, it was determined if the regenerative benefits observed in response to LSC-CM can be accounted for by EXOs. The findings indicated that LSC-EXOs are capable of reproducing part of the regenerative abilities of the CM from which they were isolated (Figs. 5-6). It was also demonstrated that LSC-EXOs outperformed MSC-EXOs in reversing pulmonary fibrosis and restoring healthy lung function. As expected all CM and EXO treated groups showed significant improvement in terms of decreased apoptotic cells, reduced fibrosis severity, and improved lung function and restoration of the alveolar epithelium. However, both LSC-CM and LSC-EXOs exhibited greater therapeutic effects as compared to both MSC-CM and -EXOs. LSC-CM was the only treatment capable of reversing the alveolar damage, reducing the fibrotic score and hydroxyproline levels to that of the sham control. In regards to pulmonary function measures, only LSC-EXO showed a significant recovery of total respiratory resistance.

RNA sequencing analysis of LSC-EXOs and MSC-EXOs showed the enrichment of miRNAs in the let-7 and miR99 families, both present in the top ten miRNAs identified in both LSC-EXOs and MSC-EXOs (Fig. 6K). The let-7 family of miRNAs was the first miRNA discovered and is highly conserved in plants and animals. Let-7 miRNA has been found to play an essential role in biological development, stem cell differentiation, and tumorigenesis. Similarly, the miR99 family (miR99a, miR99b, and miR100) of miRNAs are also highly conserved miRNAs found to be highly expressed in stem cells and have been found to be downregulated in lung injury and cancer. Different subtypes of let-7 miRNAs are found in specific tissues, cells, and cancer types. Let-7a, -7c, -7g, and miR100 were downregulated in lung cancer, and all four types are found to be in the top 10 miRNA types detected in both LSC-EXOs and MSC-EXOs.

miR99a, miR100, and miR10a have also been found to be modulated in response to DNA damage and in lungs exposed to cigarette smoke. The miR99 family of microRNAs, along with miR-151a, miR-10a and miR-30a, is significantly upregulated in LSC-EXOs compared to the other miRNAs identified. Likewise, let-7a and -7f are significantly upregulated in MSC-EXOs.

Studies have shown antifibrotic properties of miR-30a by reversing transforming growth factor-beta (TGF-β) induced Wnt1-inducible signaling pathway protein 1 (WISP1) and inhibiting mitochondria fission preventing apoptosis. WISP1 is a known profibrotic mediator in IPF patients shown to enhance ECM deposition and promoting fibrotic progression. Even though miR-30a and the let-7 and miR99 family of miRNAs are identified in both LSC- and MSC-EXOs their differential expression may explain the difference in treatment effects observed.

To assess any systemic immunogenicity or off-target effects of inhaled CM and EXO therapies, systemic cytokine expression, blood biochemistry analysis and histology of all major organs were examined (Figs. 2G, 6A). Normal tissue histology in all organs, along with normal liver and kidney functions, suggested that CM and EXO treatment did not elicit any local or systemic immune reaction and did not cause any aberrant cellular or tissue alterations. Interestingly, the analysis of systemic cytokine expression showed a downregulation of monocyte chemoattractant protein 1 (MCP-1) in blood serum of treated animals (Fig. 6A). Profibrotic cytokine MCP-1 has been reported to play a key role in lung inflammation and increase in MCP-1 levels has been linked to poor prognosis for IPF patients.

Inhalation treatment, which in some cases was administered for seven consecutive days can be delivered by such as a nebulizer for clinical relevance. Results revealed that LSC-CM and LSC-EXO treatments could attenuate and reverse bleomycin- and silica-induced fibrosis by reestablishing normal alveolar structure, decreasing extracellular matrix accumulation and myofibroblast proliferation. It has been surprisingly found that stem cell-derived conditioned media can promote alveolar repair and resolve pulmonary fibrosis at least partially through delivery via EXOs. In addition, LSC-CM and LSC-EXOs exhibited superior therapeutic benefits compared to their counterparts derived from bone marrow mesenchymal stem cells. LSC-CM and LSC-EXOs, therefore, provide advantageous therapeutic options for pulmonary fibrosis.

In some embodiments of the disclosure, the administration of the compositions to a patient in need thereof can result in a reduction of a lung pathology associated with lung fibrosis, a reduction of hydroxyproline accumulation, a reduction of expression of fibrogenic proteins, wherein the subject in need thereof has at least one of the following: a primary or secondary lung fibrotic disease. In some embodiments, the administration of the effective dose can result in an improvement of pulmonary function tests including forced vital capacities or oxygen diffusion.

In summary, we report here novel acellular therapeutic agents, namely LSC-CM and LSC-EXO that are shown to be safe and effective in the treatment of bleomycin- and silica-induced pulmonary fibrosis in rodents. The evidence presented here indicates that the mechanism of CM-mediated regeneration can relate to the EXOs, MMP2 activity, and a host of proteins found in the CM. Conditioned media and EXOs are considerably less immunogenic then their parent cells and administration of these factors can overcome the limitations of stem cells while still maintaining similar effects. The identification of miR-30a and the miR-99 and let-7 family of miRNAs in CM warrants future investigations, since miR-30a is known to be downregulated in IPF patients, and the miR-99 and let-7 families of miRNAs are also known to be downregulated in various cancers including lung cancer. Idiopathic pulmonary fibrosis is a fatal respiratory disorder with increasing rates of morbidity and mortality; with no effective therapies currently available, LSC-CM and LSC-EXO provide promising candidates for the development of IPF therapies.

### Stem cell-conditioned media attenuates and reverses bleomycin-induced fibrosis and fibroblast apoptosis

To test the impact of LSC-CM on lung repair and fibrosis, a single high dose intratracheal (i.t.) bleomycin (Bleo)-injection model in immunocompetent CD1 mice was used. Body weight was monitored throughout the study as a sign of disease burden. Since the goal was to treat fibrosis and not inflammation, the initial inflammation phase was allowed to pass and the fibrosis to become established before starting treatment. In the Bleo model inflammation peaks around day seven post instillation and transitions to fibrosis around day nine; Bleo insult began after day ten intervention (Fig. 1A).

At day ten, the mice were given inhalation treatment using a nebulizer for seven consecutive days with a dose of 10 mg of conditioned medium (CM) protein per kg of body weight or an equal volume of PBS. To verify that nebulized substances were able to reach the distal lung regions mice were tested with nebulized methylene blue dye. Lungs from one group of animals after one nebulization treatment for histological examination were immediately harvested and verified that the dye indeed reached the distal lung (Fig. 11).

At an initial macroscopic examination of therapeutic effects, all groups that received Bleo showed hemorrhagic necrosis (Fig. 1B) that was decreased with either LSC- or MSC-CM treatment. Since Bleo induces DNA damage as its molecular mechanism, the effects of CM treatment on apoptosis were examined (Figs. 1C and 1D).

LSC-CM treatment led to a reduction in apoptosis in the lung as compared to the PBS treated group. Both LSC-CM and MSC-CM treatments showed an apparent reduction in fibrosis by preserving alveolar epithelial structures (Figs. 1E and 1F) and reduced collagen deposition (Figs. 1G-1I). Histological examination of fibrosis via hematoxylin and eosin (H&E) staining and corresponding Ashcroft score revealed that only LSC-CM was able to reduce the fibrotic area and reverse the alveolar epithelial damage back to healthy levels as observed in sham controls.

### Stem cell-conditioned media inhalation treatment increases MMP2 expression and promotes vascular and alveolar repair

Lung alveoli are terminal structures of the distal airway. The alveolar epithelium is comprised of alveolar type 1 epithelial cells (AT1) that are the specialized cell type in the lungs that mediate gas exchange, and alveolar type 2 epithelial cells (AT2) that produce and release pulmonary surfactants, antioxidants, cytokines/chemokines, and other molecules important for the lung's defense, response to insult, and maintaining pulmonary homeostasis. Therefore, the changes in AT1 and AT2 distribution were examined in response to CM treatment post-Bleo injury to assess epithelial damage and rescue (Fig. 2A). Immunostaining of AT1 marker aquaporin 5 (AQP5) showed that LSC-CM treatment was able to reverse the epithelial damage caused by Bleo (Figs. 2A and 2D).

Additionally, LSC-CM treatment significantly increased the proliferation of surfactant protein C (ProSPC) positive AT2 cells (Fig. 2A and 2C) that synthesize and secrete pulmonary surfactant as well as maintaining alveolar structure and integrity by proliferating and differentiating into AT1 cells. This response still occurred with MSC-CM treatment, but at a lower level and not significantly different from the PBS treated group. Only LSC-CM treatment was able to increase the expression of von Willebrand factor (vWF) positive vasculatures in the PF lungs (Fig. 2A and 2B).

In parallel, the fibrotic response was examined by measuring protein levels of alpha-smooth muscle actin (αSMA) (Fig. 2E), which is a marker of fibroblast to myofibroblast transition in IPF. Both CM-treated groups showed a drastic decrease in αSMA when compared to PBS treated controls. Interestingly, the protein expression of matrix metalloproteinase 2 (MMP2) were increased in both CM treated groups (Fig. 2F). A cytokine array was used to access systemic cytokine expression. Only LSC-CM was able to significantly decrease IL-4 expression compared to the PBS controls (Fig. 2G).

### Therapeutic effects of conditioned media therapy in silica-induced pulmonary fibrosis

To test whether the regenerative effects of LSC-CM could be applied to other models of lung injury, the well-established silica model of induced pulmonary fibrosis was used. Unlike Bleo, a biochemical agent that causes direct cellular injury, instillation of fine silica particles into the lungs causes fibrotic nodules to develop around the particles. Again, because the goal was to treat the fibrosis and not the inflammation that precedes it in animal models, there was a delay until the inflammation phase had passed and fibrosis had set in before treating the mice. For the silica model, treatment was started at day 28 post-silica instillation and like the previous Bleo study, inhalation treatment of CM or saline was given for seven consecutive days via a nebulizer (Fig. 3A).

The physical presence of the particles embedded in the lungs aided in localization and visualization of the fibrotic response. There was no significant difference in any of the treated groups regarding effects on apoptotic cells (Fig. 3B). However, LSC-CM was able to significantly decrease the severity of the fibrosis as compared to the PBS treated group (Fig. 3C and 3D). The fibrotic tissue around the silica-induced nodules was less intense and widespread but persisted in both LSC- and MSC-CM treated groups as shown in Figs. 3C, 3E, and 3F). CM treatment also reduced collagen deposition and prevented alveolar epithelial damage as compared to the PBS treatment, although collagen deposition was still significantly higher than that of the healthy sham controls (Fig. 3F-3G).

Examination of the AQP5+ AT1 cells and ProSPC+ AT2 cells revealed a significant decline in both alveolar markers in all silica-injured lungs (Figs. 3J and 3K). However, LSC-CM was able to reduce the alveolar epithelial damage as compared to the PBS group by promoting ProSPC+ AT2 cell expression and maintaining the AQP5+ AT1 cell population (Figs. 3I and 3J). The fibrotic nodules were absent of AQP5 expressing AT1 cells, but, contained limited expression of ProSPC- and vWF-positive cells (Fig. 3K).

### Protein composition of lung spheroid cell-conditioned media

The proteomic composition of LSC-CM was defined. Pooled CM from three different donor LSC lines was used and used liquid chromatography-tandem mass spectrometry (LC/MS/MS) analysis to examine their proteome and compare them to known stem cell secretomes (Figs. 7A-7C).

The three LSC lines had similar proteomes even though the cell lines were derived from individuals of different sex, race and age (Fig. 4A). Of the shared proteins, 29.6% were annotated as extracellular proteins with known membrane receptors for secretion and 47.5% were annotated as cytoplasmic proteins with no known secretion pathways (Fig. 4B).

The high percentage of cytoplasmic proteins prompted the verification of whether the proteins identified in the CM were truly secreted proteins from the cells or were released from dead cells. Thus, the identified cytoplasmic proteins from the CM were compared with those proteins identified from lysed cells. As expected, when LSCs were lysed, the majority of the proteome consisted of annotated cytoplasmic proteins (65%) and only 5% of annotated extracellular proteins. When comparing the cytoplasmic proteins detected in the LSC-CM with the lysed cells, only 56 out of 222 (approximately 25%) of the proteins classified as cytoplasmic proteins in the CM could be from potentially lysed or dead cells. These findings suggest that the other approximately 75% of the proteins annotated as cytoplasmic proteins identified in LSC-CM are proteins that are released from the cells via a mechanism or pathway that is currently unknown.

The majority of the 103 common extracellular proteins identified in all three donors were growth factors and extracellular matrix-related proteins. Most notably from this list were proteins involved in Wnt signaling (dickkopf WNT signaling pathway inhibitor 3 [DKK3]), the complement system (complement C1r subcomponent [C1r], and decorin), angiogenesis (angiopoietin-like 2 [ANGPTL2] and extracellular matrix protein 1 [ECM1]), lung development (follistatin-like 1 [FSTL1] and nidogen 2), cell proliferation (growth arrest-specific 6 [GAS6] and insulin like growth factor binding protein 2 [IGFBP2]) and extracellular matrix (ECM) remodeling (MMP 1, 2, 3, tissue inhibitor of metalloproteinase [TIMP] 1, 2, procollagen C-endopeptidase enhancer [PCOLCE], serpin family E member 1 [SERPINE1] and secreted protein acidic and rich in cysteine [SPARC]) (Fig. 4C). Gene ontology (GO) analysis of biological processes associated with the LSC secretome identified genes important in developmental process, response to stimulus, and cellular component organization or biogenesis (Fig. 4D).

### Exosomes can partially reproduce the therapeutic effects of stem cell-conditioned media

It has been demonstrated that the therapeutic components in stem cell CM is comprised of not only soluble proteins but also extracellular vesicles (EVs), particularly exosomes (EXOs). Accordingly, the LSC-EXOs and MSC-EXOs were characterized by size, morphology and the common exosomal markers (CD63 and CD81) (Fig. 5A-5C). To test the functional effects of EXOs, a Bleo-induced PF model in SD rats was used similar to the mouse model previously presented (Fig. 5D).

All CM and EXO treated groups showed therapeutic effects in terms of maintaining normal lung architecture (Fig. 5E) and having decreased fibrosis (Fig. 5f), lung apoptosis, and collagen deposition (Figs. 5G and 5H). Notably, only LSC-CM and LSC-EXO significantly decreased collagen deposition as compared to the PBS treated group. Additionally, only LSC-CM significantly decreased the fibrosis (Ashcroft score) back to similar levels as the healthy sham control. Protein levels of αSMA showed a non-significant trend of decline in treatment groups (Fig. 5I). LSC-CM and LSC-EXO treatment attenuated alveolar epithelial and vascular injury and reduced fibrotic response, as shown by increased AQP5⁺ and vWF⁺ cells and decreased αSMA⁺ cells (Fig. 5J-5K).

### Effects of LSC-CM and LSC-EXO therapy on MMP2 and MCP-1 expression

To examine potential molecular mechanisms involved in the response to CM- and EXO- initiated responses, systemic cytokine expression and local tissue protein levels of MMP2 and SMAD3 were examined. Unlike with the murine Bleo model, no significant change in MMP2 protein levels in lung tissue of treated animals (Fig. 5I) were found. However, protein levels of SMAD3 in lung tissue of treated animals were significantly upregulated in the PBS group, which was alleviated by LSC-CM and LSC-EXO treatment. Because the immunomodulating effects of cytokines are systemic rather than local, cytokine levels in blood serum of treated animals (Fig. 6A) were examined. Monocyte chemoattractant protein-1 (MCP-1/CLL2) was significantly upregulated by Bleo injury, which was rescued by stem cell-CM and -EXO treatment.

### Effects of stem cell-conditioned media and exosome therapies on lung function following bleomycin-induced fibrosis

The clinical impact of LSC-CM and LSC-EXO treatment on impaired lung architecture and fibrosis was examined. Multiple pulmonary function tests were performed in parallel with the inhalation treatment to monitor the decline in lung function following Bleo injury and any effects of the various treatments. Baseline reads were recorded immediately before Bleo instillation, midpoint reads were taken at day nine post-Bleo (the day before treatment started), and endpoint measurements were taken at day 17 post-Bleo (one day after the last inhalation treatment) (Fig. 6B). As expected, inspiratory capacity (IC), resistance (Rrs), compliance (Crs), hysteresis area, and forced expiratory volume (FEV) to forced vital capacity (FVC) ratio all showed a decline at midpoint measurements as compared to baseline (Figs. 6C-6E, 6G, 6C-6E and 6G, respectively). Consistently, respiratory elastance (Ers), which is the inverse of compliance, consistently increased at midpoint analysis (Fig. 6F). Altogether, pulmonary function of all Bleo animals was consistently impaired as expected with pulmonary injury resulting in increased tissue stiffness and elastic recoil (elastance).

At endpoint analysis, pulmonary function was only partially rescued by CM and EXO treatment. Inspiratory capacity and respiratory compliance were both significantly improved after LSC-CM, LSC-EXO or MSC-EXO treatment. Respiratory resistance had a significant recovery only with LSC-EXO treatment. Elastance, hysteresis area and FEV/FVC ratio all had no significant changes after treatment.

### Toxicity of conditioned media and exosome therapies

In addition to the effectiveness of CM and EXO treatment, the safety of the treatments was also assessed. Liver and kidney function were found to be within an acceptable range in all treatment groups (Fig. 12). Histological analysis of the heart, kidney, liver, and spleen tissues of treated animals in both the silica and Bleo studies did not reveal any apparent damage nor toxicity (Fig. 13).

### MicroRNA-30a, miR-99a, and miR-100 are highly enriched in LSC-EXOs

Exosomes contain a variety of different RNAs, proteins, and lipids, but miRNAs are of interest since their discovery within EXOs. It is believed that EXOs utilize miRNAs as a mechanism of genetic exchange between cells. A global small RNA deep sequencing of LSC- and MSC-EXO was, therefore, performed, and the differences in their miRNA populations were analyzed.

Together, over 600 unique miRNAs were detected in these LSC-EXO and MSC-EXO samples, indicating that exosomal-derived miRNAs could have numerous regulatory roles in these samples. After removing less abundant miRNAs, 142 remained for downstream analysis (Figs. 6I and 6J). In total, 42 miRNAs were found to be differentially expressed between these two EXO types (minimum fold change of two and adjusted p-value < 0.05). Among the top upregulated miRNAs in LSC-EXO were hsa-miR-99a-5p (log2 fold change 1.25, adjusted p-value 0.0005) and hsa-miR-100-5p (log2 fold change 1.27, adjusted p-value 0.0005) (Fig. 6J). They were also the two most abundant miRNAs in LSC-EXO and are members of the miR-99 family (Fig. 6K). Antifibrotic miR-30a-3p was significantly upregulated in LSC-EXO compared to MSC-EXO (log2 fold change 2.28, adjusted p-value 0.00002). hsa-let-7a-5p (log2 fold change 1.09, adjusted p-value 0.001) and has-let-7f-5p (log2 fold change 1.28, adjusted p-value 0.008) were the most upregulated in the MSC-EXO sample and are part of the highly conserved let-7 family (Figs. 14 and 15).

Accordingly, one aspect of the disclosure encompasses embodiments of a pharmaceutical composition comprising a lung spheroid cell-conditioned medium or a lung spheroid cell-secreted therapeutic agent, or a population of lung spheroid cell-derived exosomes in an amount effective in modulating a pulmonary pathological condition when delivered to an animal or human subject in need thereof.

In some embodiments of this aspect of the disclosure, the pulmonary pathological condition is pulmonary lung fibrosis.

In some embodiments of this aspect of the disclosure, the pharmaceutical composition can comprise at least one of an isolated polypeptide, an isolated peptide, or an isolated nucleic acid, and wherein the isolated polypeptide, isolated peptide, or isolated nucleic acid is secreted by a cultured lung spheroid cell.

In some embodiments of this aspect of the disclosure, the pharmaceutical composition can comprise a population of lung spheroid cell-derived exosomes isolated from a lung spheroid cell-conditioned medium.

In some embodiments of this aspect of the disclosure, the nucleic acid can be an miRNA.

In some embodiments of this aspect of the disclosure, the pharmaceutical composition administered to the respiratory tract of the animal or human subject can further comprise a pharmaceutically acceptable carrier.

In some embodiments of this aspect of the disclosure, the pharmaceutical composition can be a lyophilized conditioned cell culture medium generated by culturing a population of lung spheroid cells therein.

In some embodiments of this aspect of the disclosure, the pharmaceutical composition administered to the respiratory tract of an animal or human subject by the nebulizer can be a lyophilized powder.

Another aspect of the disclosure encompasses embodiments of a method of producing a pharmaceutical composition comprising a lung spheroid cell-conditioned medium or a lung spheroid cell-secreted therapeutic agent, or a population of lung spheroid cell-derived exosomes, the method comprising the steps of: (a) obtaining a population of isolated pulmonary stem cells from a pulmonary tissue of an animal or human subject; (b) generating a conditioned cell culture medium by culturing the isolated population of stem cells in a cell culture medium; and (c) removing the cultured cells from the conditioned culture medium.

In some embodiments of this aspect of the disclosure, the method can further comprise the step of isolating the conditioned cell culture medium at least one of (a) at least one of an isolated polypeptide, an isolated peptide, or an isolated nucleic acid secreted by a cultured lung spheroid cell into the conditioned cell culture, or (b) a population of lung spheroid cell-derived exosomes generated by a population cultured lung spheroid cell into the conditioned cell culture medium.

In some embodiments of this aspect of the disclosure, the method can further comprise the step of lyophilizing the conditioned culture medium.

In some embodiments of this aspect of the disclosure, the method can further comprise the step of lyophilizing the at least one of (a) at least one of an isolated polypeptide, an isolated peptide, or an isolated nucleic acid secreted by a cultured lung spheroid cell into the conditioned cell culture, or (b) a population of lung spheroid cell-derived exosomes generated by a population cultured lung spheroid cell into the conditioned cell culture medium.

In some embodiments of this aspect of the disclosure, the method the lyophilized product of the method can be combined with a pharmaceutically acceptable medium.

Yet another aspect of the disclosure encompasses embodiments of a method of treating a pathological condition of an animal or human pulmonary system, wherein the method comprises administering to a region of a the respiratory tract of an animal or human subject a pharmaceutical composition comprising a lung spheroid cell-conditioned medium or a lung spheroid cell-secreted therapeutic agent, or a population of lung spheroid cell-derived exosomes in an amount effective in modulating a pulmonary pathological condition when delivered to the animal or human subject in need thereof.

In some embodiments of this aspect of the disclosure, the pulmonary pathological condition is pulmonary fibrosis.

In some embodiments of this aspect of the disclosure, the pharmaceutical composition can comprise at least one of an isolated polypeptide, an isolated peptide, or an isolated nucleic acid, and wherein the isolated polypeptide, isolated peptide, or isolated nucleic acid is secreted by a cultured lung spheroid cell.

In some embodiments of this aspect of the disclosure, the pharmaceutical composition can comprise a population of lung spheroid cell-derived exosomes isolated from a lung spheroid cell-conditioned medium.

In some embodiments of this aspect of the disclosure, the nucleic acid can be an miRNA.

In some embodiments of this aspect of the disclosure, the pharmaceutical composition administered to the respiratory tract of the animal or human subject can further comprise a pharmaceutically acceptable carrier.

In some embodiments of this aspect of the disclosure, the pharmaceutical composition can be a lyophilized conditioned cell culture medium generated by culturing a population of lung spheroid cells therein.

In some embodiments of this aspect of the disclosure, the pharmaceutical composition administered to the respiratory tract of an animal or human subject by the nebulizer can be a lyophilized powder.

It should be emphasized that the embodiments of the present disclosure, particularly any "advantageous" embodiments, are merely possible examples of the implementations, merely set forth for a clear understanding of the principles of the disclosure. Many variations and modifications may be made to the above-described embodiment(s) of the disclosure without departing substantially from the spirit and principles of the disclosure. All such modifications and variations are intended to be included herein within the scope of this disclosure, and protected by the following claims.

The specific examples below are to be construed as merely illustrative, and not limitative of the remainder of the disclosure in any way whatsoever. Without further elaboration, it is believed that one skilled in the art can, based on the description herein, utilize the present disclosure to its fullest extent. All publications recited herein are hereby incorporated by reference in their entirety.

The following examples are put forth so as to provide those of ordinary skill in the art with a complete disclosure and description of how to perform the methods and use the compositions and compounds disclosed and claimed herein. Efforts have been made to ensure accuracy with respect to numbers (*e.g*., amounts, temperature, *etc*.), but some errors and deviations should be accounted for. Unless indicated otherwise, parts are parts by weight, temperature is in °C, and pressure is at or near atmospheric. Standard temperature and pressure are defined as 20 °C and 1 atmosphere.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also include individual concentrations (*e.g.,* 1%, 2%, 3%, and 4%) and the sub-ranges (e.g., 0.5%, 1.1%, 2.2%, 3.3%, and 4.4%) within the indicated range. The term "about" can include ±1%, ±2%, ±3%, ±4%, ±5%, ±6%, ±7%, ±8%, ±9%, or ±10%, or more of the numerical value(s) being modified.

### EXAMPLES

### Example 1

*Cell Culture:* Human LSCs were generated from whole lung samples and expanded as described in Dinh *et al.,* (2017) *Respiratory Research.* Human LSCs were generated from healthy whole lung donors and expanded as described in Fig. 5. Passage 2-5 LSCs were used for all *in vitro* and *in vivo* testing. Cells were also analyzed by flow cytometry for the appropriate markers before use (CD90+, CD105+, CCSP+, AQP5+, ProSPC+, Epcam+, CD31-, CD34- and CD45-).

### Example 2

*Conditioned Media Collection and Preparation:* Human LSCs and MSCs were cultured to approximately 75% confluence before the serum-containing media was removed and replaced with serum-free media (IMDM). The following day the cells were washed six times for 30 min each with fresh IMDM to deplete the albumin on the cells before the media was allowed to condition, as albumin can interfere with some experiments (especially LC/MS/MS analysis). The media (IMDM) was allowed to condition for three days before it was harvested and filtered through a 0.22 µm filter to remove any cells and cell debris. The filtered CM was stored at -80 °C for at least 24 hrs or until solid. The frozen CM vial(s) were lyophilized using a LABCONCO FreeZone 2.5 Liter Freeze Dry System overnight or until samples were dehydrated. Once samples were lyophilized, they were stored at -20 °C until ready for use.

### Example 3

*Exosome Isolation and Characterization:* Exosomes were collected and purified from human LSC-CM using an ultrafiltration method. LSC-CM was first filtered through a 0.22 µm filter to remove any cells or cell debris. The filtered media was then placed in a 100 kDa MWCO ultrafiltration filter (Millipore) and centrifuged at 5,000 RCF for 10-15 min depending on volume. Any media contents or small proteins were removed by the filtered centrifugation, and the remaining exosomes were suspended in PBS then filtered and washed. Before use, all exosome samples were analyzed for proper size by nanoparticle tracking analysis (NTA; NanoSight, Malvern) and morphology by transmission electron microscopy (TEM). Additionally, successful exosome isolation was confirmed by immunoblotting for known exosome markers (CD63 and CD81).

### Example 4

*Animal Procedures:* Six to eight week old male CD1 mice [Crl: CD1 (ICR)] and CD (SD) IGS rats [Crl: CD(SD)] were obtained from Charles River Laboratory (Massachusetts, USA) and A/J mice were obtained from Jackson Laboratory (Maine, USA). Pulmonary fibrosis was induced by a single intratracheal injection of 3 U/kg bleomycin sulfate (EMD; 203401) solution in CD1 mice and CD (SD) rats and a single oropharyngeal aspiration of a 100 mg/kg silica (MIN-U-SIL-5) suspension in A/J mice. Nebulizer treatment started 10 days post bleomycin insult and 28 days post silica insult. Conditioned media, exosome or saline inhalation treatment was given for approximately 30 min/day for seven consecutive days using a nebulizer (Pari Trek S Portable Compressor Nebulizer Aerosol System; 047F45-LCS). Animals were euthanized, and blood and tissues were collected for RNA, protein, and histological examination.

### Example 5

*Pulmonary Function Test (PFT) in Rats:* Pulmonary function measurements were performed on the FlexiVent (SCIREQ Inc., Montreal, Canada). Prior to measurements, animals were anesthetized with an intraperitoneal injection of ketamine and xylazine solution (2:1 ratio). The animals were intubated with a 14-gauge cannula.

### Example 6

*Histology:* Immunostaining was performed on tissue slides fixed in 4% paraformaldehyde (PFA) (Electron Microscopy Sciences; 15710) followed by permeabilization and blocking with Dako Protein blocking solution (DAKO; X0909) containing 0.1% saponin (Sigma-Aldrich; 47036) prior to antibody staining. Cells were stained with antibodies against AQP5 (ab78486, Abcam), ProSPC (ab90716, Abcam), vWF (F3520, Sigma-Aldrich), αSMA (ab5694, Abcam). Tunel staining was performed on cryosectioned tissues using the In Situ Cell Death Detection Kit (Roche Diagnostics, Mannheim, Germany) according to manufacturer's instructions. Gomori's Trichrome and Hematoxylin and eosin were performed on paraffin embedded tissue sections. Hematoxylin and eosin stained sections were used for Ashcroft scoring. Ashcroft score was performed by averaging the score from one blinded and one non-blinded scorer.

### Example 7

*Proteomic Analysis:* To concentrate the samples, 15 ml of the conditioned media was lyophilized. The samples dried to completion in 24 h and were re-suspended in 1 ml of 100 mM ammonium bicarbonate (Sigma Aldrich) buffer at pH 8.6. For protein precipitation, 10 ml of cold acetone (Optima grade, ThermoFisher Scientific) was added to the samples, incubated at -20 °C overnight, and then centrifuged for 30 min at 10,000 rpm to separate the precipitated proteins from the supernatant. Total protein concentration was determined using the Bradford Assay (Pierce, ThermoFisher Scientific).

All samples were loaded in triplicate (10 µl) onto a microtiter plate, measured at an absorbance of 595 nm using a Tecan Genios microplate reader, and protein concentrations estimated using reference absorbance of BSA standard protein. Precipitated proteins were digested using the Filter Aided Sample Preparation (FASP) method as described by Wisniewski et al. In a 30 kDa molecular weight cutoff Vivacon filter (Satortorius, ThemoFisher Scientific), approximately 100 µg of protein from each sample were added, reduced with 5 mM dithiothereitol (ThermoFisher Scientific) at 56 °C for 30 min and then alkylated with 10 mM iodoacetamide (Sigma-Aldrich) for 20 min in the dark at RT. Digestion on-filter was carried out using sequencing grade trypsin (Promega) at a 1:100 trypsin-to-protein ratio overnight at 37 °C. Peptides were removed from the filter with 100 mM ammonium bicarbonate buffer, pH 8.6, and the solvent was evaporated using vacuum centrifugation before storage at -20 °C for further processing.

LC/MS/MS analysis for all samples was performed on an Easy nano ultra-high pressure liquid chromatograph coupled to an LTQ Orbitrap Elite mass spectrometer (ThermoFisher Scientific). Samples were injected onto a PepMap C18, 5 µm trapping column (ThermoFisher Scientific) then separated by in-line gradient onto a New Objective Self Pack PicoFrit column (packed in house with 3.0 µm Reprosil C18 stationary phase (Dr. Maisch GmbH). The linear gradient for separation was 5-40% mobile phase B over 90 min at 300 nl/min flow rate, where mobile phase A was 2% acetonitrile/0.1% formic acid in water and mobile phase B was 0.1% formic acid in acetonitrile. The Orbitrap Elite operated in a data-dependent mode, where the five most intense precursors were selected for subsequent fragmentation. Resolution for the precursor scan (*m*/*z* 400-2000) was set to 60,000 at *m*/*z* 400 with a target value of 1 x 10⁶ ions. The MS/MS scans were also acquired in the orbitrap with a normalized collision energy setting of 27 for HCD. For internal mass calibration, the ion of polycyclodimethylsiloxane with *m*/*z* 445.120025 was used as the lock mass. Monoisotopic precursor selection was enabled, and precursors with unknown charge or a charge state of +1 were excluded.

Raw data files were processed using Proteome Discoverer (1.4, ThermoFisher Scientific). Peak lists were searched against a forward and reverse Homo sapiens UniProt database using Mascot (1.4.1.14 Matrix Science). The parameters used for identification of tryptic peptides were: 10 ppm precursor ion mass tolerance, 0.01 Da fragment mass tolerance; up to two missed cleavage sites; carbamidomethylation of Cys was set as a fixed modification; oxidation of Met was set as a variable modification. Scaffold (4.8.4 Proteome software) was used to filter the data, quantify peptides/proteins, and perform statistical analysis. A minimum of 2 peptides per protein at a peptide and protein threshold of 95% were required for high confidence identification. Ingenuity Proteomic Analysis (IPA, QIAGEN Redwood City) was used for classification of subcellular localization of the common proteins. The listed common secreted proteins were classified using Panther (Protein Analysis Through Evolutionary Relationships) and DAVID (The Database for Annotation, Visualization and Integrated Discovery) to explore molecular function, cellular components and pathways.

### Example 9

*SDS-PAGE and Western Blot:* Samples were reduced by β-mercaptoethanol and denatured at 90°C for 5 min. Proteins were separated by gel electrophoresis carried out in triplicate on a 4-15% Tris-Glycine stain-free gel (Bio-Rad) along with a molecular weight standard (Bio-Rad, Precision Plus Protein Unstained Standards MW Ladder 161-0363). The gel was run at a stack voltage at 100V for approximately 5 min followed by a constant 200V. The gel was activated and visualized by UV light in a Bio-Rad Imager.

The Bio-Rad Mini-PROTEAN Tetra Cell system was used for a wet transfer. The SDS-PAGE gel was assembled into the apparatus with a PVDF membrane stacked between filter papers. Following the transfer, the membrane was washed three times in PBS-T for 5 min each then blocked using 5% milk in PBS-T for one hour. A 4 °C overnight incubation of primary antibody was performed for MMP2 (ab86607, Abcam), αSMA (ab5694, Abeam), SMAD3 (MA5-14939, ThermoFisher Scientific), CD63 (nb100-77913, Novus Biologicals), CD81 (MA5-13548, ThermoFisher Scientific), and GAPDH (MA5-15738-HRP, ThermoFisher Scientific) and then followed by a 1-hr incubation with the corresponding HRP conjugated secondary antibodies.

### Example 10

*Small RNA Library Construction and Sequencing:* Exosomal RNA was isolated using a commercially available total exosome RNA isolation kit (Qiagen's exoRNeasy Serum Plasma Kit). Once exosomal RNA was isolated, it was further analyzed by sequencing. RNA quantity, 260/280 ratios, and 260/230 ratios were assessed by Nanodrop (ThermoFisher). RNA integrity was verified on a Bioanalyzer 2100 using an Agilent RNA 6000 Nano Assay. Small RNA libraries were prepared according to the manufacturer's protocol using a NEBNext.RTM Multiplex Small RNA Library Prep Set for Illumina (New England Biolabs). Libraries were size selected using a Pippen Prep (Sage Science), and quality-checked by Bioanalyzer using an Agilent High Sensitivity DNA Kit. Libraries were quantified by a Quant-iT.RTM dsDNA High Sensitivity Assay Kit (ThermoFisher) and sequenced on an Illumina NextSeq500 using a mid-output V2 kit.

*Mapping and Differential Expression Analysis of miRNAs:* Raw reads were demultiplexed and quality trimmed using the standard Illumina bcl2fastq conversion software. Using mirDeep2, reads had adapters removed and were clustered using default parameters. Reads were then aligned to human rRNAs using Bowtie and the remaining unaligned reads were mapped to miRBase 22 also using Bowtie. For downstream analysis, identified miRNAs were filtered by requiring that a miRNA have at least ten read counts in at least two of the five samples. Differential expression analysis between MSC samples and LSC samples was performed using the edgeR and limma R packages and upper quantile normalized read counts. miRNAs were considered differentially expressed if the absolute log2 fold change was greater than 1 and if the adjusted p-value (FDR) was less than 0.05.

### Example 11

*Statistical Analysis:* All results are expressed as the mean ± standard deviation (SD). Two-tailed Student's t-test was used for comparison between two groups. Non-parametric one-way ANOVA (Kruskal-Wallis test) was used for comparison of three or more groups with additional Bonferroni post hoc correction. Differences were considered statistically significant at P-values ≤ 0.05. * P-values ≤ 0.05; ** P-values ≤ 0.01; *** P-values ≤ 0.001; **** P-values ≤ 0.0001.

### Example 12

**Table 1: Identified Proteins of Cultured Lung Spheroid Cell Exosomes**

| | | | |
|---|---|---|---|
| P02751; | Fibronectin | O94985; | Calsyntenin-1 |
| P08123; | Collagen alpha-2(I) | P09486; | SPARC |
| P12111; | Collagen alpha-3(VI) | Q12841; | Follistatin-related 1 |
| P02452; | Collagen alpha-1(I) | F6SYF8; | Dickkopf-related 3 |
| P11047; | Laminin subunit gamma-1 | Q14315; | Filamin-C |
| Q99715; | Collagen alpha-1(XII) | P21333; | Filamin-A |
| A0A0A0MQS9; | Laminin subunit alpha-4 | Q92626; | Peroxidasin homolog |
| P08670; | Vimentin | Q14112; | Nidogen-2 |
| P02461; | Collagen alpha-1(III) | P07996; | Thrombospondin-1 |
| G3XAI2; | Laminin subunit beta-1 | C9JIZ6; | Prosaposin |
| 000391; | Sulfhydryl oxidase 1 | O02818; | Nucleobindin-1 |
| P08253; | 72 kDa type IV collagenase | Q15113; | Procollagen C-endopeptidase enhancer 1 |
| P09871; | Complement C1s subcomponent | P14618; | Pyruvate kinase PKM |
| P12109; | Collagen alpha-1(VI) | P51884; | Lumican |
| A0A0A0MSD0; | Sushi, von Willebrand factor type A, EGF and pentraxin domain-containing 1 | P16035; | Metalloproteinase inhibitor 2 |
| | | P01033; | Metalloproteinase inhibitor 1 |
| P04264; | Keratin, type II cytoskeletal 1 | P60709; | Actin, cytoplasmic 1 |
| B4DPQ0; | Complement C1r subcomponent | Q14766; | Latent-transforming growth factor beta-binding 1 |
| B1ALD9; | Periostin | | |
| Q15582; | Transforming growth factor-beta-induced ig-h3 1 | P20908; | Collagen alpha-1(V) |
| | | P02545; | Prelamin-A/C |
| P01023; | Alpha-2-macroglobulin | Q14393; | Growth arrest-specific 6 |
| P13645; | Keratin, type I cytoskeletal 10 | P11021; | Endoplasmic reticulum chaperone BiP |
| Q14767; | Latent-transforming growth factor beta-binding 2 | P23142; | Fibulin-1 |
| | | P05121; | Plasminogen activator inhibitor 1 |
| Q02809; | Procollagen-lysine,2-oxoglutarate 5-dioxygenase 1 | P35908; | Keratin, type II cytoskeletal 2 epidermal |
| | | Q9Y240; | C-type lectin domain family 11 member A |
| Q08380; | Galectin-3-binding | P08603; | Complement factor H |
| P10909; | Clusterin | A0A3B3ITK0; | Thrombospondin-2 |
| P35527; | Keratin, type I cytoskeletal 9 | P55058; | Phospholipid transfer |
| P07237; | disulfide-isomerase | E9PGN7; | Plasma protease C1 inhibitor |
| Q12805; | EGF-containing fibulin-like extracellular matrix 1 | P35052; | Glypican-1 |
| | | P04406; | Glyceraldehyde-3-phosphate dehydrogenase |
| P07093; | Glia-derived nexin | | |
| P26022; | Pentraxin-related PTX3 | Q16270; | Insulin-like growth factor-binding 7 |
| P12110; | Collagen alpha-2(VI) | P04083; | Annexin A1 |
| P05997; | Collagen alpha-2(V) | P60174; | Triosephosphate isomerase |
| O43707; | Alpha-actinin-4 | O43852; | Calumenin |
| P36955; | Pigment epithelium-derived factor | P08294; | Extracellular superoxide dismutase [Cu-Zn] |
| P07585; | Decorin | Q07954; | Prolow-density liporeceptor-related 1 |
| P07355; | Annexin A2 | P02462; | Collagen alpha-1(IV) |
| Q16610; | Extracellular matrix 1 | P02788; | Lactotransferrin |
| P35555; | Fibrillin-1 | P14543; | Nidogen-1 |
| P12814; | Alpha-actinin-1 | P02765; | Alpha-2-HS-glyco |
| J3KPS3; | Fructose-bisphosphate aldolase | A0A2R8Y6G6; | Alpha-enolase |
| P08758; | Annexin A5 | Q9BUD6; | Spondin-2 |
| P00338; | L-lactate dehydrogenase A | P08779; | Keratin, type I cytoskeletal 16 |
| P21810; | Biglycan | P07858; | Cathepsin B |
| P13611; | Versican core | P23284; | Peptidyl-prolyl cis-trans isomerase B |
| P24043; | Laminin subunit alpha-2 | 060565; | Gremlin-1 |
| P55268; | Laminin subunit beta-2 | E7EX29; | 14-3-3 zeta/delta (Fragment) |
| P08572; | Collagen alpha-2(IV) | E7EMB3; | Calmodulin-2 |
| Q76M96; | Coiled-coil domain-containing 80 | | |

## Claims

1. A pharmaceutical composition comprising a lung spheroid cell-conditioned medium in an amount effective for modulating a pulmonary pathological condition when delivered to an animal or human subject in need thereof, and a pharmaceutically acceptable carrier.

2. The pharmaceutical composition of claim 1, wherein the pulmonary pathological condition is pulmonary lung fibrosis.

3. The pharmaceutical composition of claim 1, wherein the pharmaceutical composition comprises at least one of an isolated polypeptide, an isolated peptide, or an isolated nucleic acid.

4. The pharmaceutical composition of claim 3, wherein the isolated polypeptide, isolated peptide, and/or isolated nucleic acid is secreted by a cultured lung spheroid cell.

5. The pharmaceutical composition of claim 3, wherein the nucleic acid is an miRNA.

6. The pharmaceutical composition of claim 5, wherein the miRNA is from one of the following miRNA families: miR-30a, let-7, and miR99.

7. The pharmaceutical composition of claim 1, wherein the pharmaceutical composition is a lyophilized.

8. The pharmaceutical composition of claim 1, wherein the pharmaceutical composition is a lyophilized powder formulated for administration to the respiratory tract of an animal or human subject by a nebulizer.

9. A method of producing a pharmaceutical composition comprising a lung spheroid cell-conditioned medium, the method comprising the steps of:
(a) obtaining a population of isolated pulmonary stem cells from a pulmonary tissue of an animal or human subject;
(b) generating a conditioned cell culture medium by culturing the isolated population of stem cells in a cell culture medium; and
(c) removing the cultured cells from the conditioned culture medium.

10. The method of claim 9, further comprising the step of lyophilizing the conditioned culture medium.

11. The method of claim 10, wherein the lyophilized conditioned culture medium is combined with a pharmaceutically acceptable medium.

12. A pharmaceutical composition for use in the treatment of a pulmonary pathological condition, the pharmaceutical composition comprising a lung spheroid cell-conditioned medium, wherein the pharmaceutical composition is formulated to treat a pulmonary pathological condition when delivered to an animal or human subject in need thereof.

13. The pharmaceutical composition for use of claim 12, wherein the pulmonary pathological condition is pulmonary lung fibrosis.

14. The pharmaceutical composition for use of claim 12, wherein the pharmaceutical composition is formulated to include at least one of an isolated polypeptide, an isolated peptide, or an isolated nucleic acid.

15. The pharmaceutical composition for use of claim 14, wherein the nucleic acid is an miRNA from one of the following miRNA families: miR-30a, let-7, and miR99.
